(19) Europäisches Patentamt
European Patent Office
Office européen des brevets

(11) Veröffentlichungsnummer: **0 084 194**
**B1**

(12) # EUROPÄISCHE PATENTSCHRIFT

(45) Veröffentlichungstag der Patentschrift: **30.04.86**

(21) Anmeldenummer: **82201610.1**

(22) Anmeldetag: **16.12.82**

(51) Int. Cl.[4]: **C 09 K 19/06**, C 07 C 43/20, C 07 C 43/225, C 07 C 43/205, C 07 C 43/21, C 07 C 13/28, C 07 C 15/14, C 07 C 15/18, C 07 C 25/18, C 07 C 87/50, C 07 C 93/14 // C07C121/60, C07C121/64, C07C69/035, C07C69/24, C07C69/63, C07C121/75, C07D239/28, C07D239/30, C07D239/42

(54) **Flüssigkristallmischungen.**

(30) Priorität: **14.01.82 CH 198/82**
**19.01.82 CH 292/82**
**04.05.82 CH 2725/82**

(43) Veröffentlichungstag der Anmeldung: **27.07.83 Patentblatt 83/30**

(45) Bekanntmachung des Hinweises auf die Patenterteilung: **30.04.86 Patentblatt 86/18**

(84) Benannte Vertragsstaaten: **CH DE FR GB LI**

(56) Entgegenhaltungen:
**EP-A-0 014 885**
**EP-A-0 025 119**
**DD-A-120 426**
**DD-A-122 513**
**DE-A-2 426 160**
**DE-A-2 933 563**
**DE-A-3 040 632**
**DE-A-3 042 391**
**DE-A-3 139 130**
**DE-B-1 249 278**
**FR-A-851 131**
**GB-A-2 070 594**
**US-A-2 496 067**
**US-A-3 387 051**

(73) Patentinhaber: **Merck Patent Gesellschaft mit beschränkter Haftung, Frankfurter Strasse 250, D-6100 Darmstadt (DE)**

(72) Erfinder: **Huynh-Ba, Tuong, Dr. Chem., Stockmattstr. 63, CH-5400 Baden (CH)**
Erfinder: **Osman, Maged A., Dr. Chem., Lerchenrain 1, CH-8046 Zürich (CH)**

(56) Entgegenhaltungen: (Fortsetzung)
**US-A-3 600 445**
**US-A-4 326 087**

**EP 0 084 194 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Für Flüssigkristallanzeigen vom Typ der nematischen Drehzellen, auch TN-Displays genannt, benötigt man bekanntlich nematische Flüssigkristallmassen mit positiver Anisotropie der Dielektrizitätskonstanten, auch als positive DKA bezeichnet. Dabei ist die DKA oder $\Delta\varepsilon$ definiert als $\Delta\varepsilon = \varepsilon_{\parallel} - \varepsilon_{\perp}$, worin $\varepsilon_{\parallel}$ die Dielektrizitätskonstante (DK) parallel zur Moleküllängsachse und $\varepsilon_{\perp}$ die DK senkrecht zur Molekülachse ist.

Für den Multiplexbetrieb solcher Zellen ist überdies eine möglichst steile Kontrastkennlinie der FK-Substanz erwünscht, d. h. eine möglichst sprunghafte Veränderung der Lichtabsorptionseigenschaften bei Veränderung der anliegenden Feldspannung. Dies kann wiederum durch Optimierung von zwei Auswahlkriterien für die verfügbaren anisotropen Substanzen begünstigt werden, nämlich (a) einen möglichst kleinen Wert des Verhältnisses der Elastizitätskonstanten für Biegung und Spreizung, auch als $k_{33}/k_{11}$-Wert bezeichnet, und (b) durch einen möglichst kleinen Wert des Verhältnisses der DKA ($\Delta\varepsilon$) zur DK senkrecht zur nematischen Achse ($\varepsilon_{\perp}$), d. h. von $\Delta\varepsilon/\varepsilon_{\perp}$. Die Einzelheiten hierzu sind in der Literatur (Gharadjedagji, F. et al in Rev. Phys. Appl. 11/1976/467; Metz, A. R. in SID Digest, Techn. Papers IX/1978/70 und Alt, P. M. et al in IEEE Trans. El. Dev. Ed. 21/1974/146) zu finden.

Für die Auswahl von anisotropen Verbindungen nach den genannten Kriterien stehen gegenwärtig zwei Typen anisotroper Verbindungen zur Verfügung, die jeweils zwei oder drei Ringe enthalten und folgenden Aufbau haben:

**Typ I:**

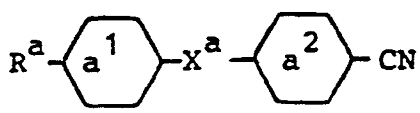

und

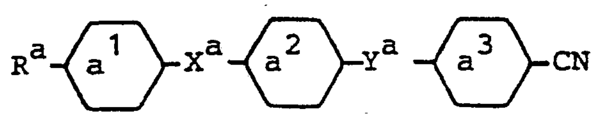

**Typ II:**

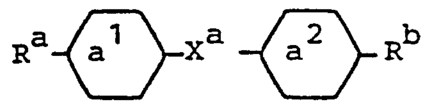

und

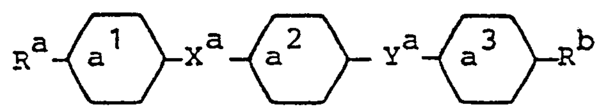

worin $a^1$, $a^2$ und $a^3$ carboaromatische, heteroaromatische oder cycloaliphatische Ringe sind, wie Benzol-, pyrimidin- und trans-Cyclohexanringe; $X^a$ und $Y^a$ sogenannte Brückengruppen, wie -COO-, -CH = N-, $-CH_2O-$, -N = N-, $-CH_2CH_2-$ oder die Einfachbindung und $R^a$, $R^b$ sogenannte Flügelgruppen, wie Alkyl-, Alkoxy-, Alkanoyloxy- oder Alkylaminogruppen sind, deren Alkylteil im allgemeinen 1 bis 12 C-Atome in gerader oder verzweigter Kette enthält.

Substanzen vom Typ I bieten wegen der vergleichsweise starken Polarisierung $R^a$/CN in Richtung der Moleküllängsachse (nematische Achse oder Direktor) hohe positive DKA-Werte und dementsprechend niedrige Schwellwertspannungen, aber mit für Multiplexbetrieb nachteilig hohen $\Delta\varepsilon/\varepsilon_\perp$-Werten und mit für diesen Zweck ebenfalls nachteiligen, vergleichsweise hohen $k_{33}/k_{11}$-Werten. Die Kontrastkennlinien dieser Substanzen sind relativ flach.

Die Substanzen vom Typ II sind wenig oder nicht polar und besitzen die für steile Kontrastkennlinien erforderlichen kleinen $\Delta\varepsilon/\varepsilon_\perp$-Werte mit im Vergleich zu Substanzen vom Typ I (bei vergleichbarer Kettenlänge) kleinen $K_{33}/k_{11}$-Werten. Ferner ist die Viskosität der Substanzen vom Typ II niedriger als die derjenigen vom Typ I.

Aus all diesen Gründen sind anisotrope Substanzen vom Typ II als Komponenten von FK-Mischungen für Multiplexbetrieb von TN-Displays unerlässlich.

Wesentlicher Nachteil der bekannten anisotropen Substanzen vom Typ II ist deren ausgeprägte Tendenz zur Bildung smektischer Phasen, insbesondere wenn diese Stoffe relativ lange Flügelgruppen, z. B. mit Alkylteilen von einiger Länge, haben. Hierzu ist auf die aus der Literatur, z. B."Flüssigkristalle in Tabellen", VEB Deutscher Verlag, Leipzig, 1974, bekannten Werte zu verweisen.

Das Auftreten von smektischen Phasen bei anisotropen Verbindungen engt den Bereich der für TN-Displays einzig geeigneten nematischen Phase ein oder bringt diese völlig zum Verschwinden.

Aufgabe der Erfindung ist es, neue anisotrope Verbindungen vom Typ II anzugeben, bei welchen die Tendenz zur Bildung smektischer Phasen zurückgedrängt bzw. ausgeschaltet ist; als Kriterium hierfür gilt, dass eine in Frage stehende Verbindung überhaupt eine nematische Phase besitzt oder dass bei vergleichbaren Strukturen die Temperaturbreite der nematischen Phase zunimmt.

Es wurde überraschenderweise gefunden, dass dies bei einer genau begrenzten Gruppe von Verbindungen des Typs II durch Einführung mindestens eines ringständigen sogenannten lateralen Substituenten in die Grundstruktur erreicht werden kann.

Die in den erfindungsgemässen Flüssigkristallmischungen verwendeten anisotropen Verbindungen vom Typ II mit nematischer Phase sind gekennzeichnet durch die Formel (1)

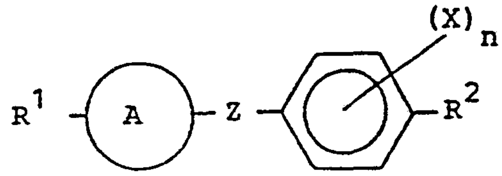

(1)

Dabei bedeutet A einen cyclischen Rest gemäss einer der Formeln (10), (11) oder (12)

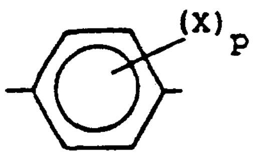

(10)

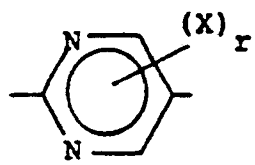

(11)

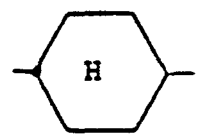

(12)

wobei die Formel (11) sowohl einen Pyrimidinring in 2,5-Ver-knüpfung als auch einen solchen in 5,2-Verknüpfung bedeuten kann.

Der cyclische Rest der Formel (12) liegt stets in der durch den Punkt angedeuteten trans-Konfiguration vor, sofern $R^1$ nicht das Wasserstoffatom ist und dementsprechend dann keine cis/trans-Isomerie besteht.

Das Brückenglied Z in Formel (1) bedeutet die Einfachbindung, die Ethylengruppe ($-CH_2CH_2-$), die Methylenoxygruppe ($-CH_2O-$) oder die Oxymethylengruppe ($-OCH_2-$), soweit nicht nachfolgend ausgeschlossen.

Die Indizes haben folgende Bedeutung: n ist 1 oder 2; p ist Null, 1 oder 2; r ist Null oder 1.

Der erfindungswesentliche, mindestens eine laterale Substituent X is Halogen, d. h. Fluor, Chlor, Brom oder Jod, oder Methyl ($-CH_3$) oder Nitril (-CN). Wenn das Molekül in Formel (1) mehrere laterale Substituenten X enthält, können diese gleich oder verschieden sein.

Dabei gelten für Verbindungen der Formel (1) folgende Massgaben:

(A) An keinen der im Molekül der Formel (1) vorhandenen aromatischen Reste der Formeln (10), (11), (13), (14) oder (15) ist eine Methylenoxy- bzw. Oxymethylengruppe direkt mit dem C-Atom einer dieser Gruppen gebunden; das Molekül der Formel (1) darf mit anderen Worten keinen Ring der Formel

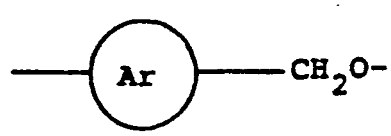

enthalten, wobei Ar einen cyclischen Rest der Formeln (10), (11), (13), (I4) oder (15) darstellt. Es wurde nämlich festgestellt, dass derartige Benzyläther und entsprechende Verbindungen mit einer Gruppe der Formel

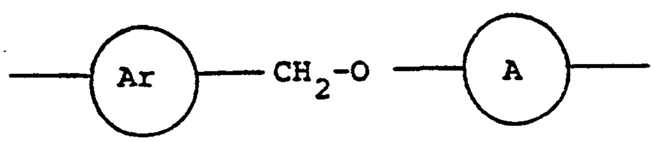

worin Ar und A die oben angegebene Bedeutung haben, bei Verwendung als oder in Flüssigkristalle(n) zur Instabilität neigen.

(B) Ferner gilt für neue Verbindungen der Formel (1) die Massgabe, dass mindestens ein X im Molekül der Formel (1) Methyl ist, wenn nur eine oder keine der Gruppen $R^1$, $R^2$ einen cyclischen Rest der Formeln (13) oder (16) bedeutet, dabei A die Formel (12) hat und das Brückenglied Z sowie das gegebenenfalls vorhandene Brückenglied $Z^3$ oder $Z^6$ die Einfachbindung darstellen,

Für viele Zwecke wird ferner bevorzugt, dass der cyclische Rest der Formel (12) bzw. dass die cyclischen Gruppen der Formel (16) jeweils nicht gleichzeitig direkt mit einem Sauerstoffatom einerseits und mit einem Sauerstoff- oder Stickstoffatom andererseits direkt verbunden sind.

Schliesslich wird für besonders stabile Verbindungen der Formel (1) bevorzugt, dass die aromatischen Ringe nicht zwi schen zwei direkt ringgebundenen Sauerstoffatomen stehen, insbesndere wenn an diesem Ring ein lateraler Substituent X = CN hängt.

$R^1$ und $R^2$ können gleich oder verscheiden sein und bedeuten Wasserstoffatome, Alkyl, Alkoxy, Alkanoyloxy (Alk-C(O)-O-) oder Alkylamino (Alk-N(H)-) mit jeweils 1 bis 12 C-Atomen im Alkylteil; der Alkylteil kann gerade oder verzweigt und gegebenenfalls chiral sein; $R^1$ oder/und $R^2$ kann ferner eine cyclische Gruppe der Formeln (13) bis (16) bedeuten:

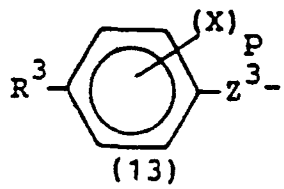

(13)

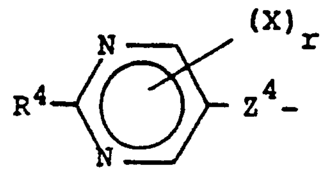

(14)

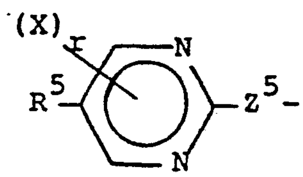

(15)

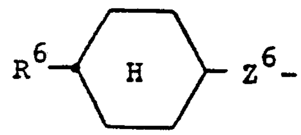

(16)

In den Formel (13) bis (16) haben die Flügelgruppen $R^3$ bis $R^6$ die oben für die nicht-cyclischen $R^1$ und $R^2$ genannten Bedeutungen; X, p und r sind wie oben angegeben und die Brückengruppen $Z^3$ bis $Z^6$ haben eine der für Z angegebenen Bedeutungen, wobei in einem Molekül der Formel (1) die Bedeutungen von Z einerseits und $Z^3$ bis $Z^6$ andererseits gleich oder verschieden sein können.

Die Verbindungen der Formel (1) können allgemein zweikernig, dreikernig oder vierkernig sein, bevorzugte neue erfindungsgemässe Verbindungen haben die in den Patentansprüchen 2-13 angegebenen und definierten Formeln (22) bis (33).

Erfindungsgemäss sind die Flüssigkristallmischungen, die mindestens eine Verbindung der Formel (1), z. B. eine oder mehrere Verbindungen der Formeln (22) bis (33) enthalten. Dies wird weiter unten noch erläutert.

Bei einer Gruppe bevorzugter Verbindungen gemäss der Erfindung bedeutet X die Methylgruppe. Bei einer anderen Gruppe bevorzugter Verbindungen gemäss der Erfindung bedeutet X Fluor, Chlor, Brom oder Jod, wobei Fluor und Chlor besonders bevorzugt werden.

Bei einer weiteren bevorzugten Gruppe von Verbindungen gemäss der Erfindung bedeutet X die Nitrilgruppe.

Wenn die Verbindung der Formel (1) ein starkes laterales Dipolmoment hat, was z. B. die Folge von zwei oder mehr X als Nitril oder/und Halogen sein kann, wird sie in der Regel auch ein stark negatives $\Delta\varepsilon$ haben und kann dann z. B. für Flüssigkristallanzeigen verwendet werden, die nach dem sogenannten inversen Guest/Host-Effekt arbeiten oder für bestimmte Typen von dynamischen Streuzellen, DAP- und HN-Anzeigen verwendet werden.

Erfindungsgemässe Verbindungen enthalten mindestens einen lateralen Substituenten X, können aber auch zwei oder mehr laterale Substitenten X aufweisen, wobei das jeweilige Molekül (1) gleiche oder aber unterschiedliche laterale Substituenten X tragen kann, z. B. Methyl plus Halogen, Methyl plus Nitril, Halogen plus Nitril oder Methyl plus Halogen plus Nitril.

Den Verbindungen der Formel (1) liegt die Erkenntnis zugrunde, dass es zur Vermeidung bzw. Zurückdrängung der Tendenz zur Bildung smektischer Phasen bei bestimmten Verbindungen vom Typ II wesentlich ist, dass der laterale Substituent bzw. die lateralen Substituenten eine gewisse aber nicht zu grosse Voluminosität aufweisen muss bzw. müssen; die Untergrenze der Voluminosität von erfindungsgemäss geeigneten lateralen Substituenten ist von der Tendenz der Grundstruktur II (d. h. Formel (1) ohne laterale Substituenten X) zur Bildung smektischer Phasen abhängig. Im allgemeinen führt die Einführung eines lateralen oder mehrerer lateraler Substituenten X mit grösserem Volumen im Vergleich zur entsprechenden Grundstruktur ohne lateralen Substituent zu einer Erniedrigung des Klärpunktes; für zweikernige Verbindungen der Formel (1) werden daher die weniger voluminösen lateralen Substituenten Fluor, Chlor und Methyl bevorzugt. Das Volumen der Substituenten X nimmt annähernd in folgender Reihe zu:

$F < Cl < CH_3 \approx Br < J < CN$

Zur Herstellung der Verbindungen (1) sind insbesondere zwei Synthesewege geeignet, nämlich einerseits die

Einführung des oder der gewünschten Substituenten X in die entsprechende Grundverbindung, z. B. durch Chlorierung oder Bromierung, gegebenenfalls mit nachfolgendem Ersatz eines Substituenten X, wie Chlor, durch einen anderen, wie Fluor, durch Halogenaustausch bzw. durch Umsetzung einer entsprechenden Halogenverbindung mit Metallcyanid zur Einführung der Nitrilgruppe. In analoger Weise kann in die entsprechende Grundverbindung eine Carbonylgruppe, z. B. durch katalysierte Umsetzung mit einem Formylierungsmittel, wie Oxalylchlorid, eingeführt und dann zur Methylgruppe reduziert werden.

Der andere Syntheseweg beruht darauf, dass zum Aufbau der Verbindung (1) die bereits mit entsprechenden Substituenten X versehenen Teilverbindungen verwendet und z. B. durch Kondensation vereinigt werden.

Ferner kann man auch zur Modifikation der Flügelgruppen $R^1$ bis $R^6$ eine erfindungsgemässe Verbindung nach an sich bekannten Methoden in eine andere erfindungsgemässe Verbindung umwandeln.

Geeignete Grund- bzw. Ausgangsverbindungen sind bekannt oder können nach analogen Methoden wie die bekannten Verbindungen erhalten werden. Geeignete Ausgangsverbindungen mit Pyrimidinkernen sind z. B. beschrieben in DD-PS 95'892 und DE-OS 26 41 724.

Als allgemeine Beispiele für die Herstellung der neuen anisotropen Verbindungen werden die nachfolgenden Reaktionsschemata gegeben, in welchen die allgemeinen Zeichen die in Formel (1) angegebene Bedeutung haben.

**SCHEMA A**

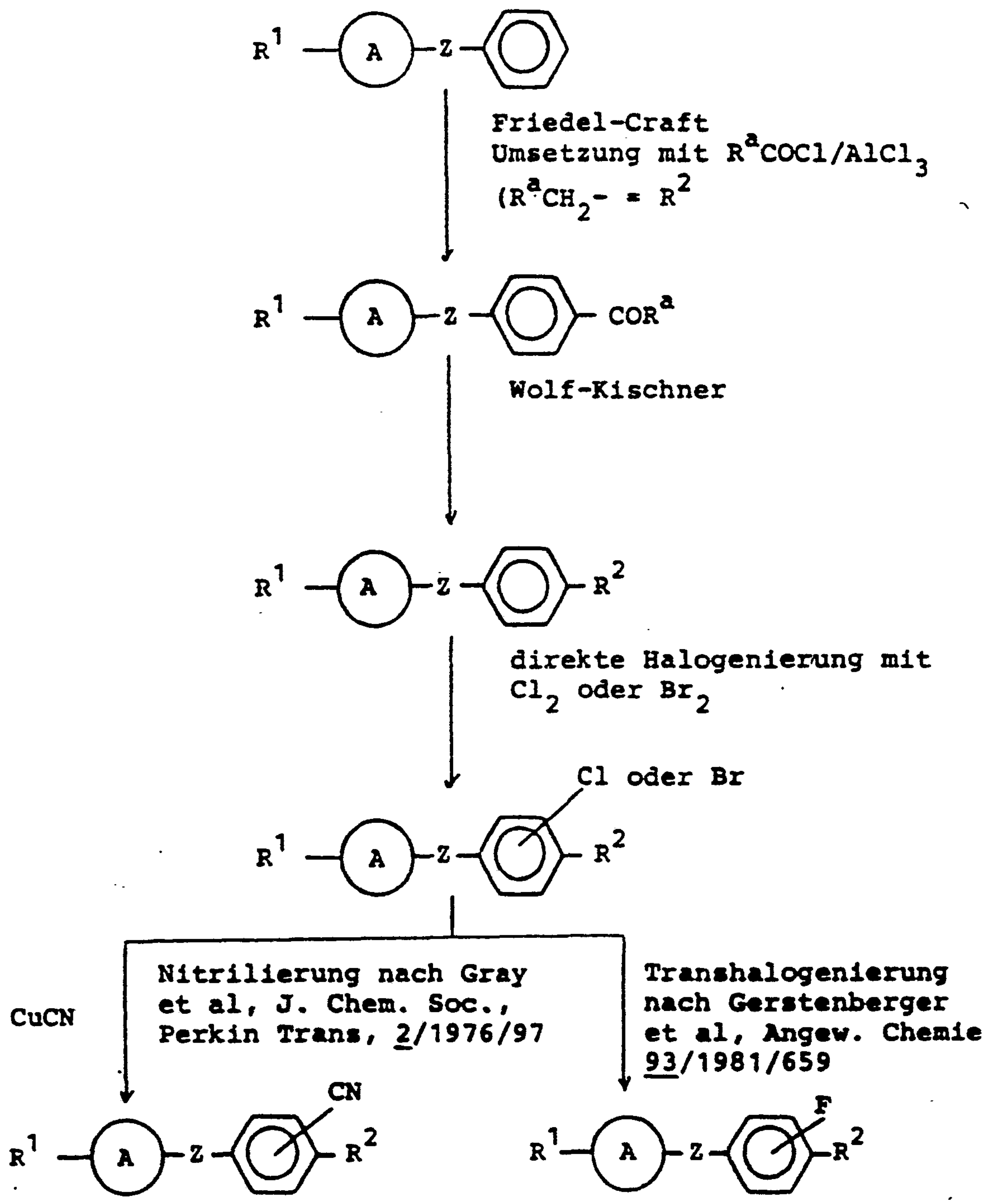

SCHEMA B

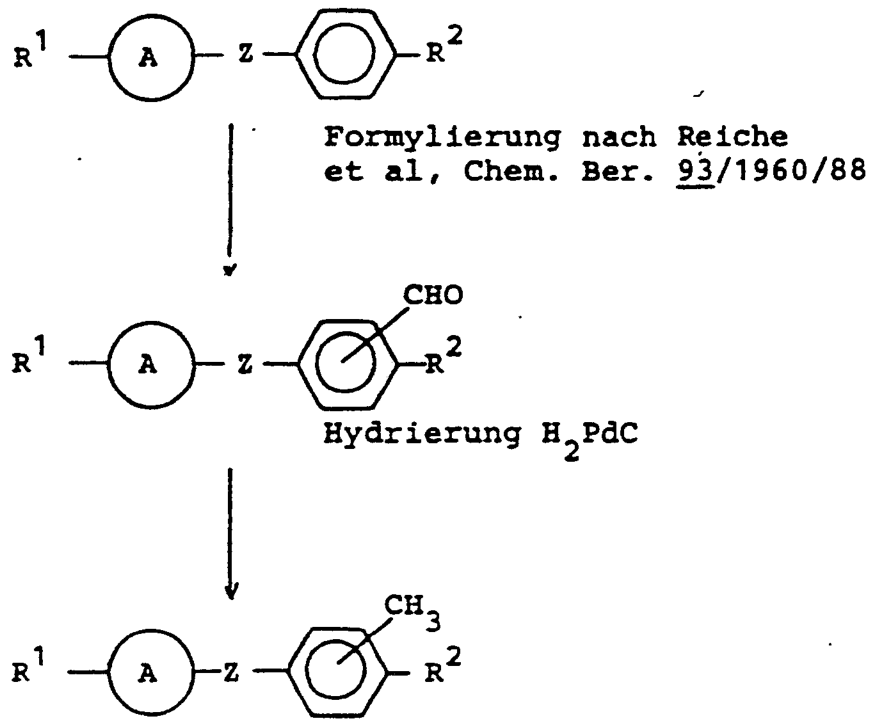


SCHEMA C

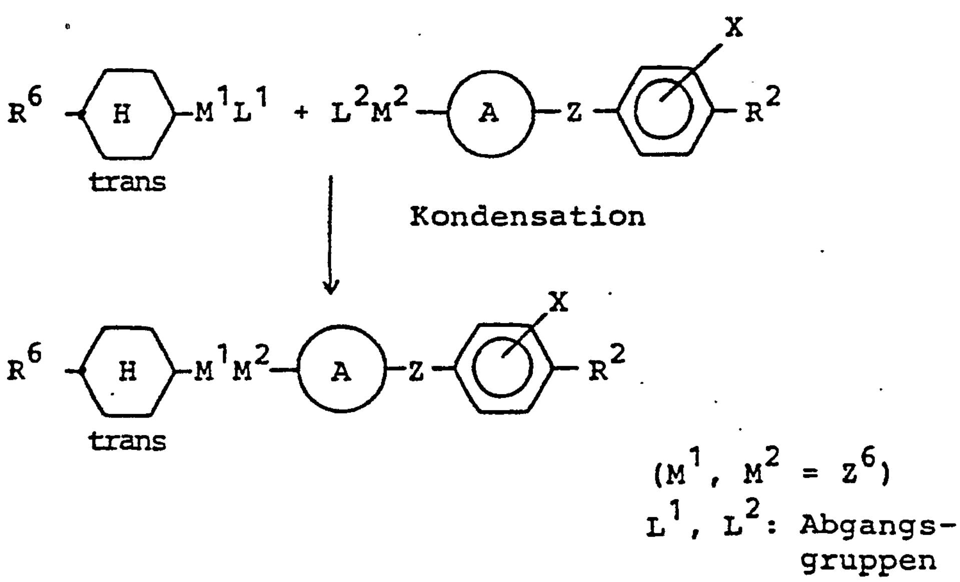

**SCHEMA D**

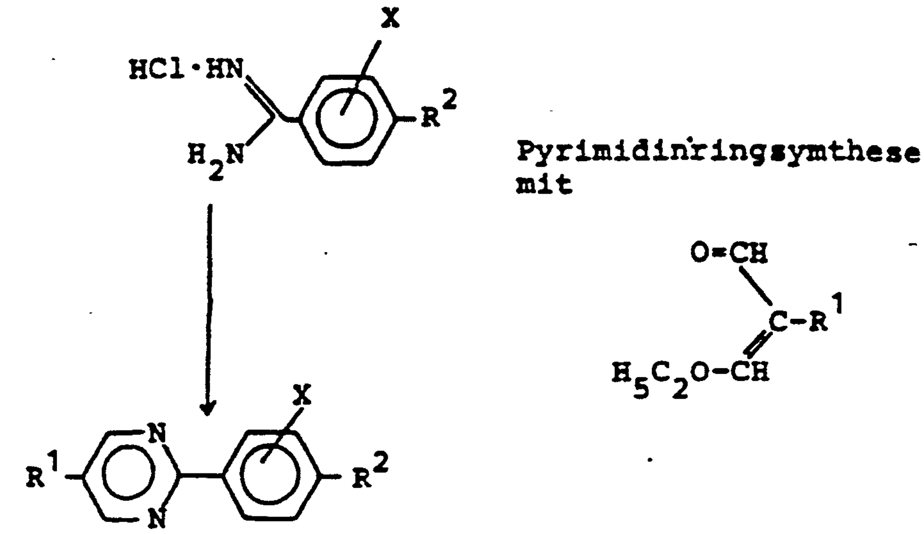


**SCHEMA E**

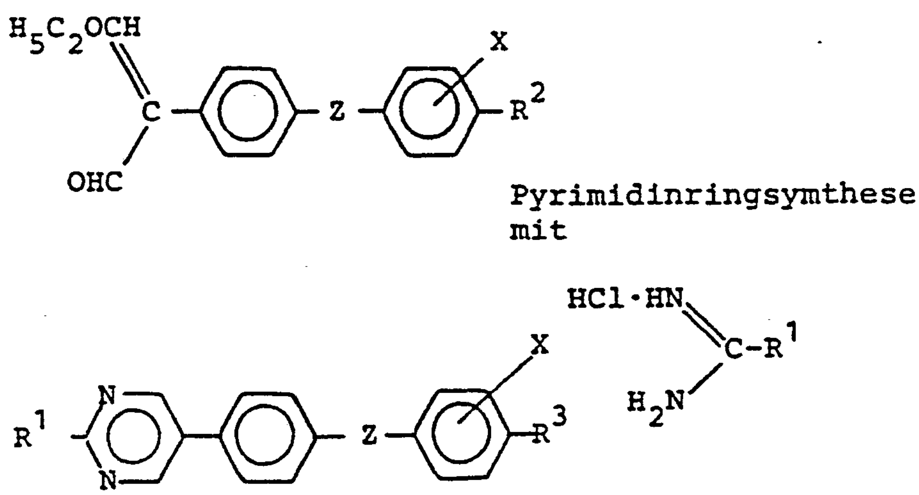


Die Verbindungen der Formel (1) können in analoger Weise wie die bekannten Verbindungen vom Typ II für FK-Mischungen in TN-Displays mit Multiplexbetrieb verwendet werden und bieten dabei den Vorteil, dass die Probleme smektischer Phasen ausgeschaltet bzw. wesentlich vermindert werden können.

Erfindungsgemässe FK-Mischungen können eine oder mehrere Verbindung(en) der Formel (1), z. B. in Anteilen von insgesamt bis etwa 60 Mol% enthalten, wobei der Anteil der einzelnen Verbindungen der Formel (1) bis etwa 30 Mol% der Mischung ausmacht. Als restlichen Anteil enthalten die erfindungsgemässen FK-Mischungen meist eine oder vorzugsweise mehrere bekannte Verbindung(en) vom Typ I.

Der weiteren Erläuterung der Erfindung dienen die nachfolgenden Beispiele. Die Temperatureigenschaften der Mesophasen sind in üblicher Weise durch die entsprechenden Temperaturwerte (°C) zwischen den Symbolen K (kristallin), S (smektisch), N (nematisch) und I (isotrop) angegeben.

**Beispiel 1**

4-Hydroxy-4'-pentyl-diphenyl wurde mit elementarem Chlor zu 3-Chlor-4-hydroxy-4'-pentyldiphenyl umgesetzt und durch Kondensation mit n-Hexyljodid in eine Verbindung der Formel (1), nämlich 3-Chlor-4-hexyloxy-4'-pentylbiphenyl der Formel (100)

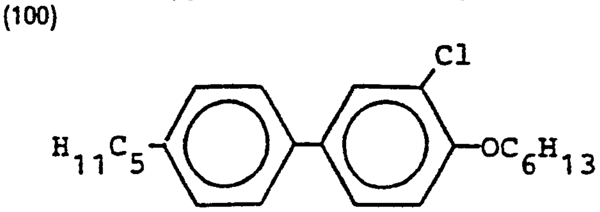
(100)

umgewandelt.

In analoger Weise, jedoch ohne Chlorierung, wurde die Vergleichsverbindung der Formel (101)

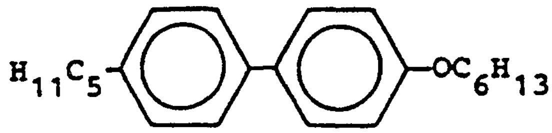

(101)

hergestellt. Die Untersuchung der Mesophasentemperaturen ergab den überraschenden Befund, dass die Verbindung der Formel (100) mit K 1.7 N 11.1 I eine rein nematische Mesophase zeigte, während die Vergleichsverbindung der Formel (101) mit K 82 S 84 I keine nematische Phase aufwies und im ganzen Mesophasenbereich smektisch war.

**Beispiel 2**

Nach der in Beispiel 1 angegebenen Methode wurde eine Verbindung der Formel (200)

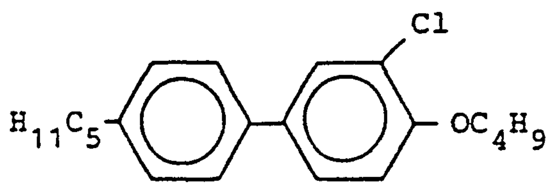

(200)

hergestellt und mit der entsprechenden Verbindung der Formel (201)

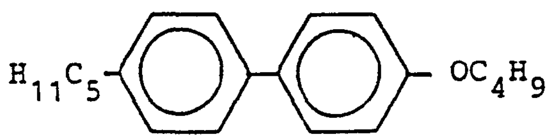

(201)

verglichen.

Die Verbindung (200) erwies sich als monotrop nematisch (K 38.1 N (7.5) I), wahrend die Vergleichsverbindung (201) nur smektische Mesophasen (K 37 S 80.1 S 88.1 I) hatte.

**Beispiel 3**

Die durch Veresterung des 3-Chlor-4-hydroxy-4'-pentyldiphe-nyl hergestellte Verbindung der Formel(300)

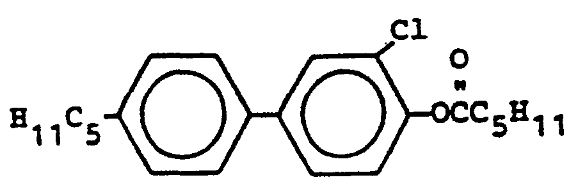

(300)

erwies sich als monotrop nematisch (K 22.3 N (-5) I), während die Verbindung der Formel (301)

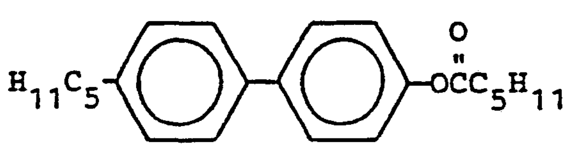

(301)

eine nur smektische Mesophase zeigte (K 45.7 S 87.4 I).

**Beispiel 4** Die neue anisotrope Verbindung der Formel (400)

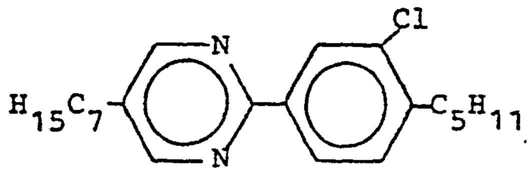

(400)

zeigte keine smektische Phase, während die Vergleichsverbindung der Formel (401)

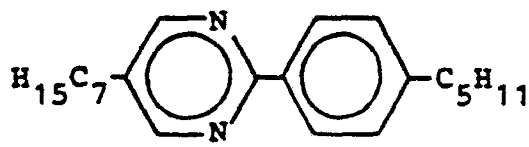

(401)

eine ausschliesslich smektische Mesophase (K 30.6 S 47.7 I) besass.

**Beispiel 5**

Die neue anisotrope Verbindung der Formel (500)

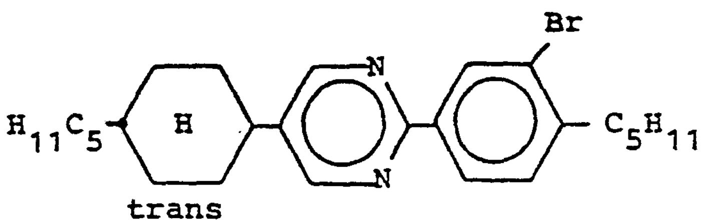

(500)

zeigte keine smektische, während die Vergleichsverbindung der Formel (501)

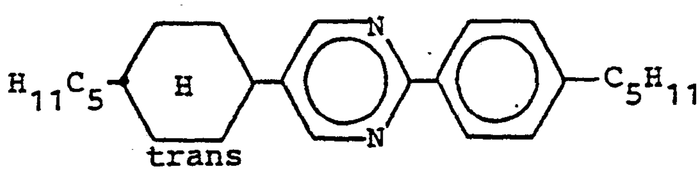

(501)

eine ausschliesslich smektische Mesophase (K 39.5 S 189.2 I) hatte.

**Beispiel 6**

Die neue anisotrope Verbindung der Formel (600)

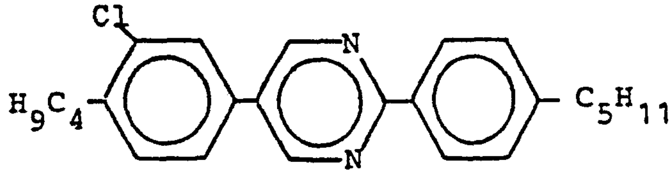

(600)

zeigte keine smektische Phase (K 111.2 N (103) I), während die Vergleichsverbindung der Formel (601)

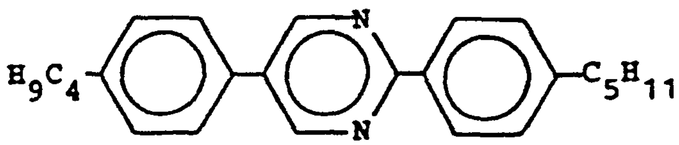

(601)

eine ausschliesslich smektische Mesophase (K 106.4 S 185.2 I) hatte.

**Beispiel 7**

Eine kalte (etwa 0° C) Mischung von 16 g Oxalylchlorid, 45 g 1,4-bis-(4-trans-Pentylcyclohexyl)-benzol und 16 g wasserfreiem Aluminiumchlorid in 100 ml sym. Tetrachlorethan wurde in einem Autoklaveinsatz gerührt, bis die Temperatur der Mischung etwa Raumtemperatur erreicht hatte (10-20 min). Dann wurden 10 g wasserfreies Natriumacetat und 1 g Pd/C-Katalysator zugesetzt. Nach Verdrängen der Luft durch Stickstoff wurde Wasserstoff unter Rühren der Reaktionsmischung bis zu einem Ueberdruck von 30 bar eingeführt. Nach ca. 120 min bei diesem Druck wurde der Autoklavinhalt auf 50° C erwärmt. Nach 30 min bei dieser Temperatur wurde das Reaktionsgemisch auf Raumtemperatur gekühlt und darauf der Wasserstoffüberdruck abgeblasen, der Autoklav mit Stickstoff gespült und geöffnet.

Die Reaktionsmischung wurde mit Wasser, nötigenfalls unter Kühlung, gewaschen und die abgetrennte organische Phase vom Lösungsmittel befreit. Der Rückstand wurde umkristallisiert. Das erhaltene Produkt der Formel (700)

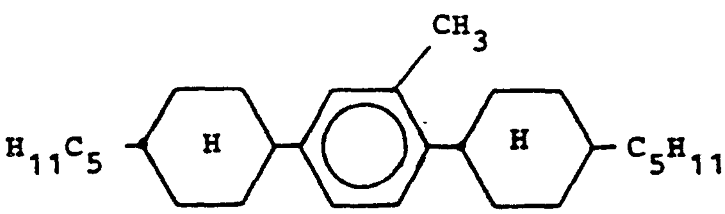

(700)

hatte einen Schmelzpunkt von 55 5° C und zeigte eine nematische Phase bis 86,5° C.

**Beispiele 8**

Die beiden Verbindungen
2'-Fluoro-4-butyloxy-4'-pentyl-diphenyl
und
3'-Fluoro-4-butyloxy-4'-pentyl-diphenyl
zeigen eine signifikante nematische Phase, während die entsprechenden Verbindungen ohne Fluor in 2'- bzw. 3'-Stellung keine signifikante nematische Phase aufweisen.

Als weitere Verbindungen der Formel (1) können die folgenden Verbindungen genannt werden:
2-Fluor-4,4'-dipropyl-diphenyl
2-Fluor-4,4'-dipentyl-diphenyl
3-Fluor-4,4'-dipropyl-diphenyl
3-Fluor-4,4'-dipentyl-diphenyl
2-Fluor-4-propyl-4'-pentyl-diphenyl
2'-Fluor-4-propyl-4'-pentyl-diphenyl
3-Fluor-4-propyl-4'-pentyl-diphenyl
3'-Fluor-4-propyl-4'-pentyl-diphenyl
2-Fluor-4-pentyl-4'-heptyl-diphenyl
2'-Fluor-4-pentyl-4'-heptyl-diphenyl
3-Fluor-4-pentyl-4'-heptyl-diphenyl
3'-Fluor-4-pentyl-4'-heptyl-diphenyl
2-Chlor-4-pentyl-4'-heptyl-diphenyl
2'-Chlor-4-pentyl-4'-heptyl-diphenyl
3-Chlor-4-pentyl-4'-heptyl-diphenyl
3'-Chlor-4-pentyl-4'-heptyl-diphenyl
3-Fluor-4-butyloxy-4'-pentyl-diphenyl
3-Fluor-4-hexyloxy-4'-pentyl-diphenyl
3-Fluor-4-butyloxy-4'-heptyl-diphenyl
3-Fluor-4-hexyloxy-4'-heptyl-diphenyl
3-Chlor-4-propanoyloxy-4'-heptyl-diphenyl
3-Chlor-4-propanoyloxy-4'-pentyl-diphenyl
3-Chlor-4-propanoyloxy-4'-nonyl-diphenyl

3-Chlor-4-pentanoyloxy-4'-heptyl-diphenyl
3-Fluor-4-butylamino-4'-pentyl-diphenyl
3-Fluor-4-hexylamino-4'-pentyl-diphenyl
3-Fluor-4-butylamino-4'-heptyl-diphenyl
2,5-Difluor-4,4'-dipentyl-diphenyl
2,5-Difluor-4,4'-diheptyl-diphenyl
2,5-Difluor-4,4'-dinonyl-diphenyl
2,5-Dichlor-4,4'-dinonyl-diphenyl
2,5-Difluor-4-pentyl-4'-heptyl-diphenyl
2,5-Difluor-4-heptyl-4'-pentyl-diphenyl
3,5-Difluor-4-butyloxy-4'-pentyl-diphenyl
3,5-Difluor-4-hexyloxy-4'-pentyl-diphenyl
3,5-Difluor-4-hexyloxy-4'-heptyl-diphenyl
3,3'-Difluor-4,4'-dibutyloxy-diphenyl
3,3'-Difluor-4-butyloxy-4'-hexyloxy-diphenyl
3,3'-Dichlor-4,4'-dihexyloxy-diphenyl
3,3'-Dichlor-4-hexyloxy-4'-octyloxy-diphenyl
3,3'-Difluor-4,4'-di(butylamino)-diphenyl
3,3'-Dichlor-4,4'-di(hexylamino)-diphenyl
1-(2-Fluor-4-pentylphenyl)-2-(4-pentylphenyl)-ethan
1-(3-Fluor-4-pentylphenyl)-2-(4-pentylphenyl)-ethan
1-(2-Chlor-4-heptylphenyl)-2-(4-heptylphenyl)-ethan
1-(3-Chlor-4-heptylphenyl)-2-(4-heptylphenyl)-ethan
1-(3-Fluor-4-butyloxyphenyl)-2-(4-heptylphenyl)-ethan
1-(3-Chlor-4-hexyloxyphenyl)-2-(4-heptylphenyl)-ethan
1,2-Bis-(2-fluor-4-pentylphenyl)-ethan
1,2-Bis-(3-fluor-4-pentylphenyl)-ethan
1,2-Bis-(3-fluor-4-butyloxyphenyl)-ethan
1,2-Bis-(3-fluor-4-hexyloxyphenyl)-ethan
1,2-Bis-(3-chlor-4-hexyloxyphenyl)-ethan
1-(3,5-Difluor-4-butyloxyphenyl)-2-(4-pentylphenyl)-ethan
1-(3-Fluor-4-pentanoyloxyphenyl)-2-(4-pentylphenyl)-ethan
1-(3,5-Difluor-4-pentanoyloxyphenyl)-2-(4-pentylphenyl)-ethan
2-Fluor-4-propyl-4'-(4-pentylphenyl)-diphenyl
2-Fluor-4-pentyl-4'-(4-pentylphenyl)-diphenyl
3-Fluor-4-pentyl-4'-(4-pentylphenyl)-diphenyl
2-Chlor-4-pentyl-4'-(4-pentylphenyl)-diphenyl
3-Chlor-4-pentyl-4'-(4-pentylphenyl)-diphenyl
2-Brom-4-pentyl-4'-(4-pentylphenyl)-diphenyl
3-Brom-4-pentyl-4'-(4-pentylphenyl)-diphenyl
2-Jod-4-pentyl-4'-(4-pentylphenyl)-diphenyl
3-Jod-4-pentyl-4'-(4-pentylphenyl)-diphenyl
2-Cyan-4-pentyl-4'-(4-Pentylphenyl)-diphenyl
3-Cyan-4-pentyl-4'-(4-pentylphenyl)-diphenyl
2-Methyl-4-pentyl-4'-(4-pentylphenyl)-diphenyl
3-Methyl-4-pentyl-4'-(4-pentylphenyl)-diphenyl
3-Fluor-4-butyloxy-4'-(4-pentylPhenyl)-diphenyl
3-Chlor-4-butyloxy-4'-(4-pentylPhenyl)-diPhenyl
3-Brom-4-butyloxy-4'-(4-Pentylphenyl)-diPhenyl
3-Jod-4-butyloxy-4'-(4-pentylphenyl)-diphenyl
3-Cyan-4-butyloxy-4'-(4-pentylphenyl)-diphenyl
3-Methyl-4-butyloxy-4'-(4-pentylphenyl)-diphenyl
3-Fluor-4-butyloxy-4'-(3-fluor-4-butyloxyphenyl)-diphenyl
3-Chlor-4-butyloxy-4'-(3-chlor-4-butyloxyphenyl)-diphenyl
3-Brom-4-butyloxy-4'-(3-brom-4-butyloxyphenyl)-diphenyl
3-Jod-4-butyloxy-4'-(3-jod-4-butyloxyphenyl)-diphenyl
3-Methyl-4-butyloxy-4'-(3-methyl-4-butyloxyphenyl)-diphenyl
2-Fluor-1,4-bis-(4',4''-dipentylphenyl)-benzol,
K 51,5 $S_B$ 62 $S_A$ 109,5 N 136,5 I
2-Chlor-1,4-bis-(4',4''-dipentylphenyl)-benzol
2-Brom-1,4-bis-(4',4''-dipentylphenyl)-benzol
2-Jod-1,4-bis-(4',4''-dipentylphenyl)-benzol
2-Cyan-1,4-bis-(4',4''-dipentylphenyl)-benzol
2-Methyl-1,4-bis-(4',4''-dipentylphenyl)-benzol
2,5-Difluor-1,4-bis-(4',4''-dipentylPhenyl)-benzol

2,5-Dichlor-1,4-bis-(4',4''-dipentylphenyl)-benzol
2,5-Dibrom-1,4-bis-(4',4''-dipentylphenyl)-benzol
2,5-Dijod-1,4-bis-(4',4''-dipentylphenyl)-benzol
2,5-Dimethyl-1,4-bis-(4',4''-dipentylphenyl)-benzol
1-(2-Fluor-4-pentylphenyl)-2-(4'-pentyldiphenyl)-ethan
1-(3-Fluor-4-pentylphenyl)-2-(4'-pentyldiphenyl)-ethan
1-(2-Chlor-4-pentylphenyl)-2-(4'-pentyldiphenyl)-ethan
1-(3-Chlor-4-pentylphenyl)-2-(4'-pentyldiphenyl)-ethan
1-(3-Brom-4-pentylphenyl)-2-(4'-pentyldiphenyl)-ethan
1-(3-Cyano-4-pentylphenyl)-2-(4'-pentyldiphenyl)-ethan
1-(2-Fluor-4-pentylphemyl)-2-(3-fluor-4'-pentyldiphenyl)-ethan
1-(2-Fluor-4-pentylphenyl)-2-(3'-fluor-4'-pentyldiphenyl)-ethan
1,4-Bis-[β-(4-pentyl-2-fluorphenyl)-ethyl]-benzol
1,4-Bis-[β-(4-pentyl-3-fluorphenyl)-ethyl]-benzol
1,4-Bis-[β-(4-pentyl-2-chlorphenyl)-ethyl]-benzol
1,4-Bis-[β-(4-pentyl-3-chlorphenyl)-ethyl]-benzol
1,4-Bis-[β-(4-pentyl-2-bromphenyl)-ethyl]-benzol
1,4-Bis-[β-(4-pentyl-3-bromphenyl)-ethyl]-benzol
1,4-Bis-[β-(4-butyloxy-2-fluorphenyl)-ethyl]-benzol
1,4-Bis-[β-(4-butyloxy-3-fluorphenyl)-ethyl]-benzol
1,4-Bis-[β-(4-butyloxy-3-chlorphenyl)-ethyl]-benzol
1-(4'-Pentylphenyl)-2-[2-fluor-4-(4'-trans-pentylcyclohexyl)-phenyl]-ethan
1-(4'-Pentylphenyl)-2-[2-chlor-4-(4'-trans-pentylcyclohexyl)-phenyl]-ethan
1-(4'-Pentylphenyl)-2-[2-brom-4-(4'-trans-pentylcyclohexyl)-phenyl]-ethan
1-(4'-Pentylphenyl)-2-[3-fluor-4-(4'-trans-pentylcyclohexyl)-phenyl]-ethan
1-(4'-Pentylphenyl)-2-[3-chlor-4-(4'-trans-pentylcyclohexyl)-phenyl]-ethan
1-(4'-Pentylphenyl)-2-[3-brom-4-(4'-trans-pentylcyclohexyl)-phenyl]-ethan
1-(4'-Pentylphenyl)-2-[3-cyan-4-(4'-trans-pentylcyclohexyl)-phenyl]-ethan
1-(4'-Pentylphenyl)-2-[3-methyl-4-(4'-trans-pentylcyclohexyl)-phenyl]-ethan
1-(4-Butyloxyphenyl)-2-[2-cyan-4-(4'-trans-pentylcyclohexyl)-phenyl]-ethan
1-(4-Butyloxyphenyl)-2-[3-cyan-4-(4'-trans-pentylcyclohexyl)-phenyl]-ethan
1-(4-Butyloxy-3-fluorphenyl)-2-[2-fluor-4-(4'-trans-pentyl-cyclohexyl)-phenyl]-ethan
1-(4-Butyloxy-3-chlorphenyl)-2-[2-chlor-4-(4'-trans-pentyl-cyclohexyl)-phenyl]-ethan
1-(-Butyloxy-3-fluorphenyl)-2-[3-fluor-4-(4'-trans-pentyl cyclohexyl)-phenyl]-ethan
1-(4-Butyloxy-3-chlorphenyl)-2-[3-chlor-4-(4'-trans-pentyl-cyclohexyl)-phenyl]-ethan
1-(4-Butyloxy-3-bromphenyl)-2-[3-brom-4-(4'-trans-pentyl-cyclohexyl)-phenyl]-ethan
1-(4-Butyloxy-3-methylphenyl)-2-[3-methyl-4-(4'-trans-pentyl-cyclohexyl)-phenyl]-ethan
1-(4-Pentylphenyl)-2-[2-fluor-4-(4'-trans-pentylcyclohexyl-methoxy)-phenyl]-ethan
1-(4-Pentylphenyl)-2-[3-fluor-4-(4'-trans-pentylcyclohexyl-methoxy)-phenyl]-ethan 41-(5-Pentylphenyl)-2-[2-chlor-4-(4'-trans-pentylcyclohexyl-methoxy)-phenyl]-ethan
1-(4-Pentylphenyl)-2-[3-chlor-4-(4'-trans-pentylcyclohexyl-methoxy)-phenyl]-ethan
1-(4-trans-Pentylcyclohexyl)-2-(2-fluor-4'-pentyldiphenyl)-ethan, Schmelzpunkt 56°, Klärpunkt 110°
1-(4-trans-Pentylcyclohexyl)-2-(2-chlor-4'-pentyldiphenyl)-ethan
1-(4-trans-Pentylcyclohexyl)-2-(2-brom-4'-pentyldiphenyl)-ethan
1-(4-trans-Pentylcyclohexyl)-2-(2-jod-4'-pentyldiphenyl)-ethan
1-(4-trans-Pentylcyclohexyl)-2-(2-methyl-4'-pentyldiphenyl)-ethan
1-(4-trans-Pentylcyclohexyl)-2-(2-cyan-4'-pentyldiphenyl)-ethan
1-(4-trans-Pentylcyclohexyl)-2-(2,2'-difluor-4'-pentyldiphe-nyl)-ethan, $\Delta n = 0,15$
1-(4-trans-Pentylcyclohexyl)-2-(2,2'-dichlor-4'-pentyldiphe-nyl)-ethan
1-(4-trans-Pentylcyclohexyl)-2-(3,3'-difluor-4'-pentyldiphe-nyl)-ethan
1-(4-trans-Pentylcyclohexyl)-2-(3,3'-dichlor-4'-pentyldiphe-nyl)-ethan
1-(4-trans-Pentylcyclohexyl)-2-(3-fluor-4'-pentyldiphenyl)-ethan, $\Delta n = 0,15$
1-(4-trans-Pentylcyclohexyl)-2-(3-chlor-4'-pentyldiphenyl)-ethan
1-(4-trans-Pentylcyclohexyl)-2-(3-methyl-4'-pentyldiphenyl)-ethan
1-(4-trans-Pentylcyclohexyl)-2-(3-cyan-4'-pentyldiphenyl)-ethan
1-(4-trans-Pentylcyclohexyl)-2-(3,3'-difluor-4'-pentyldi-phenyl)-ethan $\Delta n = 0,14$
1-(4-trans-Pentylcyclohexyl)-2-(3,3'-dichlor-4'-pentyldi-phenyl)-ethan
1-(4-trans-Pentylcyclohexyl)-2-(2'-fluor-4'-pentyldiphenyl)-ethan K 17 S 33 $S_A$ 37 N 108,5 I
1-(4-trans-Pentylcyclohexyl)-2-(2'-chlor-4'-pentyldiphenyl)-ethan
1-(4-trans-Pentylcyclohexyl)-2-(3'-fluor-4'-pentyldiphenyl)-ethan, $\Delta n = 0,15$
1-(4-trans-Pentylcyclohexyl)-2-(3'-chlor-4'-pentyldiphenyl)-ethan
1-(4-trans-Pentylcyclohexyl)-2-(3'-methyl-4'-pentyldiphenyl)-ethan
1-(4-trans-Pentylcyclohexyl)-2-(3'-cyan-4'-pentyldiphenyl)-ethan
1-(4-trans-Pentylcyclohexyl)-2-(3'-fluor-4'-butyloxydiphe-nyl)-ethan, $\Delta n = 0,16$
1-(4-trans-Pentylcyclohexyl)-2-(3'-chlor-4'-butyloxydiphe-nyl)-ethan

1-(4-trans-Pentylcyclohexyl)-2-(3'-brom-4'-butyloxydiphenyl)-ethan
1-(4-trans-Pentylcyclohexyl)-2-(3'-jod-4'-butyloxydiphenyl)-ethan
1-(4-trans-Pentylcyclohexyl)-2-(3'-cyan-4'-butyloxydiphenyl)-ethan
1-(4-trans-Pentylcyclohexyl)-2-(3'-methyl-4'-butyloxydiphe-nyl)-ethan
1-(4-trans-Pentylcyclohexylethyl)-2-(3 -fluor-4'-butyloxy-diphenyl)-ethan
1-(4-trans-Pentylcyclohexylethyl)-2-(3'-chlor-4'-butyloxy-diphenyl)-ethan
1-(4-trans-Pentylcyclohexylethyl)-2-(3'-fluor-4'-propanoyl-oxydiphenyl)-ethan
5-Propyl-2-(2-fluor-4-propylphenyl)-pyrimidin
5-Pentyl-2-(2-fluor-4-propylphenyl)-pyrimidin
5-Pentyl-2-(2-fluor-4-pentylphenyl)-pyrimidin
5-Heptyl-2-(2-fluor-4-pentylphenyl)-pyrimidin
5-Pentyl-2-(3-fluor-4-pentylphenyl)-pyrimidin, K 28 N, Δn = 0,18
5-Heptyl-2-(3-fluor-9-heptylphenyl)-pyrimidin
5-Pentyl-2-(2-chlor-4-heptylphenyl)-pyrimidin
5-Heptyl-2-(3-chlor-4-heptylphenyl)-pyrimidin
5-Pentyl-2-(3-fluor-4-butyloxyphenyl)-pyrimidin
5-Heptyl-2-(3-fluor-4-butyloxyphenyl)-pyrimidin
5-Heptyl-2-(3-fluor-4-hexyloxyphenyl)-pyrimidin, K 56 N, Δn = 0,17
5-Pentyl-2-(3-chlor-4-butyloxyphenyl)-pyrimidin
5-Heptyl-2-(3-chlor-4-butyloxyphenyl)-pyrimidin
5-Heptyl-2-(3-chlor-4-hexyloxyphenyl)-pyrimidin
5-Pentyl-2-(3-fluor-4-propanoyloxyphenyl)-pyrimidin
5-Pentyl-2-(3-fluor-4-pentanoyloxyphenyl)-pyrimidin
5-Heptyl-2-(3-fluor-4-pentanoyloxyphenyl)-pyrimidin
5-Pentyl-2-(3-chlor-4-pentanoyloxyphenyl)-pyrimidin
5-Heptyl-2-(3-chlor-4-pentanoyloxyphenyl)-pyrimidin
2-Propyl-5-(2-fluor-4-propylphenyl)-pyrimidin
2-Pentyl-5-(2-fluor-4-propylphenyl)-pyrimidin
2-Pentyl-5-(2-fluor-4-pentylphenyl)-pyrimidin
2-Heptyl-5-(2-fluor-4-pentylphenyl)-pyrimidin
2-Pentyl-5-(3-fluor-4-pentylphenyl)-pyrimidin
2-Heptyl-5-(3-fluor-4-heptylphenyl)-pyrimidin
2-Pentyl-5-(2-chlor-4-heptylphenyl)-pyrimidin
2-Heptyl-5-(3-chlor-4-heptylphenyl)-pyrimidin
2-Pentyl-5-(3-fluor-4-butyloxyphenyl)-pyrimidin
2-Heptyl-5-(3-fluor-4-butyloxyphenyl)-pyrimidin
2-Heptyl-5-(3-fluor-4-hexyloxyphenyl)-pyridimin
2-Pentyl-5-(3-chlor-4-butyloxyphenyl)-pyrimidin
2-Heptyl-5-(3-chlor-4-butyloxyphenyl)-pyrimidin
2-Heptyl-5-(3-chlor-4-hexyloxyphenyl)-pyrimidin
2-Pentyl-5-(3-fluor-4-propanoyloxyphenyl)-pyrimidin
2-Pentyl-5-(3-fluor-4-pentanoyloxyphenyl)-pyrimidin
2-Heptyl-5-(3-fluor-4-pentanoyloxyphenyl)-pyrimidin
2-Pentyl-5-(3-chlor-4-pentanoyloxyphenyl)-pyrimidin
2-Heptyl-5-(3-chlor-4-pentanoyloxyphenyl)-pyrimidin
1-(5-Pentyl-2-pyrimidinyl)-2-(2-fluor-4-pentylphenyl)-ethan
1-(5-Pentyl-2-pyrimidinyl)-2-(3-fluor-4-pentylphenyl)-ethan
1-(5-Pentyl-2-pyrimidinyl)-2-(2-chlor-4-pentylphenyl)-ethan
1-(5-Pentyl-2-pyrimidinyl)-2-(3-chlor-4-pentylphenyl)-ethan
1-(5-Pentyl-2-pyrimidinyl)-2-(3-fluor-4-butyloxyphenyl)-ethan
1-(5-Pentyl-2-pyrimidinyl)-2-(3-chlor-4-butyloxyphenyl)-ethan
1-(5-Pentyl-2-pyrimidinyl)-2-(3-fluor-4-propanoyloxyphenyl)-ethan
1-(5-Pentyl-2-pyrimidinyl)-2-(3-chlor-4-propanoyloxyphenyl)-ethan
5-(4-Pentylphenyl)-2-(2-fluor-4-pentylphenyl)-pyrimidin
5-(4-Pentylphenyl)-2-(3-fluor-4-pentylphenyl)-pyrimidin
5-(4-Pentylphenyl)-2-(3-chlor-4-pentylphenyl)-pyrimidin
5-(4-Pentylphenyl)-2-(3-fluor-4-butyloxyphenyl)-pyrimidin
5-(4-Pentylphenyl)-2-(3-chlor-4-butyloxyphenyl)-pyrimidin
5-(4-Pentylphenyl)-2-(3-fluor-4-propanoyloxyphenyl)-pyr-imidin
2,5-Bis-(2-fluor-4-pentylphenyl)-pyrimidin
2,5-Bis-(3-fluor-4-pentylphenyl)-pyrimidin
2,5-Bis-(3-chlor-4-pentylphenyl)-pyrimidin
2,5-Bis-(3-fluor-4-butyloxyphenyl)-pyrimidin
2,5-Bis-(3-chlor-4-butyloxyphenyl)-pyrimidin
2,5-Bis-(3-fluor-4-propanoyloxyphenyl)-pyrimidin

2,5-Bis-(3-chlor-4-propanoyloxyphenyl)-pyrimidin
1-(2-Pentyl-5-pyrimidinyl)-2-(2-fluor-4-pentylphenyl)-ethan
1-(2-Pentyl-5-pyrimidinyl)-2-(3-fluor-4-pentylphenyl)-ethan
1-(2-Pentyl-5-pyrimidinyl)-2-(2-chlor-4-pentylphenyl)-ethan
1-(2-Pentyl-5-pyrimidinyl)-2-(3-chlor-4-pentylphenyl)-ethan
1-(2-Pentyl-5-pyrimidinyl)-2-(3-fluor-4-butyloxyphenyl)-ethan
1-(2-Pentyl-5-pyrimidinyl)-2-(3-chlor-4-butyloxyphenyl)-ethan
1-(2-Pentyl-5-pyrimidinyl)-2-(3-fluor-4-propanoyloxyphenyl)-ethan
1-(2-Pentyl-5-pyrimidinyl)-2-(3-chlor-4-propanoyloxyphenyl)-ethan
2-(4-Pentylphenyl)-5-(2-fluor-4-pentylphenyl)-pyrimidin
2-(4-Pentylphenyl)-5-(3-fluor-4-pentylphenyl)-pyrimidin
2-(4-Pentylphenyl)-5-(3-chlor-4-pentylphenyl)-pyrimidin
2-(4-Pentylphenyl)-5-(3-fluor-4-butyloxyphenyl)-pyrimidin
2-(4-Pentylphenyl)-5-(3-chlor-4-butyloxyphenyl)-pyrimidin
2-(4-Pentylphenyl)-5-(3-fluor-4-propanoyloxyphenyl)-pyrimidin
2-Pentyl-5-(2-fluor-4'-pentyldiphenyl)-pyrimidin
2-Pentyl-5-(2-chlor-4'-pentyldiphenyl)-pyrimidin
2-Pentyl-5-(2-brom-4'-pentyldiphenyl)-pyrimidin
2-Pentyl-5-(3-fluor-4'-pentyldiphenyl)-pyrimidin
2-Pentyl-5-(3-chlor-4'-pentyldiphenyl)-pyrimidin
2-Pentyl-5-(3-brom-4'-pentyldiphenyl)-pyrimidin
2-Pentyl-5-(3-cyan-4'-pentyldiphenyl)-pyrimidin
2-Pentyl-5-(3'-fluor-4'-butyloxydiphenyl)-pyrimidin
2-Pentyl-5-(3'-chlor-4'-butyloxydiphenyl)-pyrimidin
2-Pentyl-5-(3'-brom-4'-butyloxydiphenyl)-pyrimidin
2-Pentyl-5-(3'-cyan-4'-butyloxydiphenyl)-pyrimidin
2-Pentyl-5-(3'-methyl-4'-butyloxydiphenyl)-pyrimidin
2-Pentyl-5-(3'-chlor-4'-propanoyloxydiphenyl)-pyrimidin
5-Pentyl-2-[2-fluor-4-(4-pentylphenylethyl)-phenyl]-pyrimidin
5-Pentyl-2-[2-chlor-4-(4-pentylphenylethyl)-phenyl]-pyrimidin
5-Pentyl-2-[2-brom-4-(4-pentylphenylethyl)-phenyl]-pyrimidin
5-Pentyl-2-[2-cyan-4-(4-pentylphenylethyl)-phenyl]-pyrimidin
5-Pentyl-2-(2-fluor-4'-pentyldiphenyl)-pyrimidin
5-Pentyl-2-(2-chlor-4'-pentyldiphenyl)-pyrimidin
5-Pentyl-2-(2-brom-4'-pentyldiphenyl)-pyrimidin
5-Pentyl-2-(3-fluor-4'-pentyldiphenyl)-pyrimidin
5-Pentyl-2-(3-chlor-4'-pentyldiphenyl)-pyrimidin
5-Pentyl-2-(3-brom-4'-pentyldiphenyl)-pyrimidin
5-Pentyl-2-(3-cyan-4'-pentyldiphenyl)-pyrimidin
5-Pentyl-2-(3'-fluor-4'-butyloxydiphenyl)-pyrimidin
5-Pentyl-2-(3'-chlor-4'-butyloxydiphenyl)-pyrimidin
5-Pentyl-2-(3'-brom-4'-butyloxydiphenyl)-pyrimidin
5-Pentyl-2-(3'-cyan-4'-butyloxydiphenyl)-pyrimidin
5-Pentyl-2-(3'-methyl-4'-butyloxydiphenyl)-pyrimidin
5-Pentyl-2-(3'-chlor-4'-propanoyloxydiphenyl)-pyrimidin
2-Pentyl-5-[2-fluor-4-(4-pentylphenylethyl)-phenyl]-pyrimidin
2-Pentyl-5-[2-chlor-4-(4-pentylphenylethyl)-phenyl]-pyrimidin
2-Pentyl-5-[2-brom-4-(4-pentylphenylethyl)-phenyl]-pyrimidin
2-Pentyl-5-[2-cyan-4-(4-pentylphenylethyl)-phenyl]-pyrimidin
5-(trans-4-Pentyltyclohexyl)-2-(2-fluor-4-pentylphenyl)-pyrimidin
5-(trans-4-Pentylcyclohexyl)-2-(2-chlor-4-pentylphenyl)-pyrimidin
5-(trans-4-Pentylcyclohexyl)-2-(2-brom-4-pentylphenyl)-pyrimidin
5-(trans-4-Pentylcyclohexyl)-2-(2-cyan-4-pentylphenyl)-pyrimidin
5-(trans-4-Pentylcyclohexyl)-2-(2-methyl-4-pentylphenyl)-pyrimidin
5-(trans-4-Pentylcyclohexyl)-2-(3-fluor-4-pentylphenyl)-pyrimidin
5-(trans-4-Pentylcyclohexyl)-2-(3-chlor-4-pentylphenyl)-pyrimidin
5-(trans-4-Pentylcyclohexyl)-2-(3-brom-4-pentylphenyl)-pyrimidin
5-(trans-4-Pentylcyclohexyl)-2-(3-cyan-4-pentylphenyl)-pyrimidin
5-(trans-4-Pentylcyclohexyl)-2-(3-methyl-4-pentylphenyl)-pyrimidin
5-(trans-4-Pentylcyclohexyl)-2-(3-fluor-4-butyloxyphenyl)-pyrimidin
5-(trans-4-Pentylcyclohexyl)-2-(3-chlor-4-butyloxyphenyl)-pyrimidin
5-(trans-4-Pentylcyclohexyl)-2-(3-brom-4-butyloxyphenyl)-pyrimidin
5-(trans-4-Pentylcyclohexyl)-2-(3-cyan-4-butyloxyphenyl)-pyrimidin
5-(trans-4-Pentylcyclohexyl)-2-(3-fluor-4-propanoyloxyphe-nyl)-pyrimidin
5-(trans-4-Pentylcyclohexyl)-2-(3-chlor-4-propanoyloxyphe-nyl)-pyrimidin

5-(trans-4-Pentylcyclohexyl)-2-(3-brom-4-propanoyloxyphe-nyl)-pyrimidin
5-(trans-4-Pentylcyclohexyl)-2-(3-cyan-4-propanoyloxyphe-nyl)-pyrimidin
2-(trans-4-Pentylcyclohexyl)-5-(2-fluor-4-pentylphenyl)-pyrimidin
2-(trans-4-Pentylcyclohexyl)-5-(2-chlor-4-pentylphenyl)-pyrimidin
2-(trans-4-Pentylcyclohexyl)-5-(2-brom-4-pentylphenyl)-pyrimidin
2-(trans-4-Pentylcyclohexyl)-5-(2-cyan-4-pentylphenyl)-pyrimidin
2-(trans-4-Pentylcyclohexyl)-5-(2-methyl-4-pentylphenyl)-pyrimidin
2-(trans-4-Pentylcyclohexyl)-5-(3-fluor-4-pentylphenyl)-pyrimidin
2-(trans-4-Pentylcyclohexyl)-5-(3-chlor-4-pentylphenyl)-pyrimidin
2-(trans-4-Pentylcyclohexyl)-5-(3-brom-4-pentylphenyl)-pyrimidin
2-(trans-4-Pentylcyclohexyl)-5-(3-cyan-4-pentylphenyl)-pyrimidin
2-(trans-4-Pentylcyclohexyl)-5-(3-methyl-4-pentylphenyl)-pyrimidin
2-(trans-4-Pentylcyclohexyl)-5-(3-fluor-4-butyloxyphenyl)-pyrimidin
2-(trans-4-Pentylcyclohexyl)-5-(3-chlor-4-butyloxyphenyl)-pyrimidin
2-(trans-4-Pentylcyclohexyl)-5-(3-brom-4-butyloxyphenyl)-pyrimidin
2-(trans-4-Pentylcyclohexyl)-5-(3-cyan-4-butyloxyphenyl)-pyrimidin
2-(trans-4-Pentylcyclohexyl)-5-(3-fluor-4-propanoyloxyphe-nyl)-pyrimidin
2-(trans-4-Pentylcyclohexyl)-5-(3-chlor-4-propanoyloxyphe-nyl)-pyrimidin
2-(trans-4-Pentylcyclohexyl)-5-(3-brom-4-propanoyloxyphe-nyl)-pyrimidin
2-(trans-4-Pentylcyclohexyl)-5-(3-cyan-4-propanoyloxyphe-nyl)-pyrimidin
5-(trans-4-Pentylcyclohexylethyl)-2-(2-fluor-4-pentylphe-nyl)-pyrimidin
5-(trans-4-Pentylcyclohexylethyl)-2-(2-chlor-4-pentylphe-nyl)-pyrimidin
5-(trans-4-Pentylcyclohexylethyl)-2-(2-brom-4-pentylphenyl)-pyrimidin
5-(trans-4-Pentylcyclohexylethyl)-2-(2-cyan-4-pentylphenyl)-pyrimidin
5-(trans-4-Pentylcyclohexylethyl)-2-(2-methyl-4-pentylphe-nyl)-pyrimidin
5-(trans-4-Pentylcyclohexylethyl)-2-(3-fluor-4-pentylphe-nyl)-pyrimidin
5-(trans-4-Pentylcyclohexylethyl)-2-(3-chlor-4-pentylphe-nyl)-pyrimidin
5-(trans-4-Pentylcyclohexylethyl)-2-(3-brom-4-pentylphenyl)-pyrimidin
5-(trans-4-Pentylcyclohexylethyl)-2-(3-cyan-4-pentylphenyl)-pyrimidin
5-(trans-4-Pentylcyclohexylethyl)-2-(3-methyl-4-pentylphe-nyl)-pyrimidin
5-(trans-4-Pentylcyclohexylethyl)-2-(3-fluor-4-butyloxy-phenyl)-pyrimidin
5-(trans-4-Pentylcyclohexylethyl)-2-(3-chlor-4-butyloxy-phenyl)-pyrimidin
5-(trans-4-Pentylcyclohexylethyl)-2-(3-brom-4-butyloxyphe-nyl)-pyrimidin
5-(trans-4-Pentylcyclohexylethyl)-2-(3-cyan-4-butyloxyphe-nyl)-pyrimidin
5-(trans-4-Pentylcyclohexylethyl)-2-(3-fluor-4-propanoyl-oxyphenyl)-pyrimidin
5-(trans-4-Pentylcyclohexylethyl)-2-(3-chlor-4-propanoyl-oxyphenyl)-pyrimidin
5-(trans-4-Pentylcyclohexylethyl)-2-(3-brom-4-propanoyloxy-phenyl)-pyrimidin
5-(trans-4-Pentylcyclohexylethyl)-2-(3-cyan-4-propanoyloxy-phenyl)-pyrimidin
2-(trans-4-Pentylcyclohexylethyl)-5-(2-fluor-4-pentyl-phenyl)-pyrimidin
2-(trans-4-Pentylcyclohexylethyl)-5-(2-chlor-4-pentyl-phenyl)-pyrimidin
2-(trans-4-Pentylcyclohexylethyl)-5-(2-brom-4-pentyl-phenyl)-pyrimidin
2-(trans-4-Pentylcyclohexylethyl)-5-(2-cyan-4-pentyl-phenyl)-pyrimidin
2-(trans-4-Pentylcyclohexylethyl)-5-(2-methyl-4-pentyl-phenyl)-pyrimidin
2-(trans-4-Pentylcyclohexylethyl)-5-(3-fluor-4-pentyl-phenyl)-pyrimidin
2-(trans-4-Pentylcyclohexylethyl)-5-(3-chlor-4-pentyl-phenyl)-pyrimidin
2-(trans-4-Pentylcyclohexylethyl)-5-(3-brom-4-pentyl-phenyl)-pyrimidin
2-(trans-4-Pentylcyclohexylethyl)-5-(3-cyan-4-pentyl-phenyl)-pyrimidin
2-(trans-4-Pentylcyclohexylethyl)-5-(3-methyl-4-pentyl-phenyl)-pyrimidin
2-(trans-4-Pentylcyclohexylethyl)-5-(3-fluor-4-butyloxy-phenyl)-pyrimidin
2-(trans-4-Pentylcyclohexylethyl)-5-(3-chlor-4-butyloxy-phenyl)-pyrimidin
2-(trans-4-Pentylcyclohexylethyl)-5-(3-brom-4-butyloxy-phenyl)-pyrimidin
2-(trans-4-Pentylcyclohexylethyl)-5-(3-cyan-4-butyloxy-phenyl)-pyrimidin
2-(trans-4-Pentylcyclohexylethyl)-5-(3-fluor-4-propanoyl-oxyphenyl)-pyrimidin
2-(trans-4-Pentylcyclohexylethyl)-5-(3-chlor-4-propanoyl-oxyphenyl)-pyrimidin
2-(trans-4-Pentylcyclohexylethyl)-5-(3-brom-4-propanoyl-oxyphenyl)-pyrimidin
2-(trans-4-Pentylcyclohexylethyl)-5-(3-cyan-4-propanoyl-oxyphenyl)-pyrimidin
5-(trans-4-Pentylcyclohexylmethoxy)-2-(2-fluor-4-pentyl-phenyl)-pyrimidin
5-(trans-4-Pentylcyclohexylmethoxy)-2-(2-chlor-4-pentyl-phenyl)-pyrimidin
5-(trans-4-Pentylcyclohexylmethoxy)-2-(2-brom-4-pentyl-phenyl)-pyrimidin
5-(trans-4-Pentylcyclohexylmethoxy)-2-(2-cyan-4-pentyl-phenyl)-pyrimidin
5-(trans-4-Pentylcyclohexylmethoxy)-2-(2-methyl-4-pentyl-phenyl)-pyrimidin
5-(trans-4-Pentylcyclohexylmethoxy)-2-(3-fluor-4-pentyl-phenyl)-pyrimidin
5-(trans-4-Pentylcyclohexylmethoxy)-2-(3-chlor-4-pentyl-phenyl)-pyrimidin
5-(trans-4-Pentylcyclohexylmethoxy)-2-(3-brom-4-pentyl-phenyl)-pyrimidin
5-(trans-4-Pentylcyclohexylmethoxy)-2-(3-cyan-4-pentyl-phenyl)-pyrimidin

5-(trans-4-Pentylcyclohexylmethoxy)-2-(3-methyl-4-pentyl-phenyl)-pyrimidin
5-(trans-4-Pentylcyclohexylmethoxy)-2-(3-fluor-4-butyloxy-phenyl)-pyrimidin
5-(trans-4-Pentylcyclohexylmethoxy)-2-(3-chlor-4-butyloxy-phenyl)-pyrimidin
5-(trans-4-Pentylcyclohexylmethoxy)-2-(3-brom-4-butyloxy-phenyl)-pyrimidin
5-(trans-4-Pentylcyclohexylmethoxy)-2-(3-cyan-4-butyloxy-phenyl)-pyrimidin
5-(trans-4-Pentylcyclohexylmethoxy)-2-(3-fluor-4-propanoyl-oxyphenyl)-pyrimidin
5-(trans-4-Pentylcyclohexylmethoxy)-2-(3-chlor-4-propanoyl-oxyphenyl)-pyrimidin
5-(trans-4-Pentylcyclohexylmethoxy)-2-(3-brom-4-propanoyl-oxyphenyl)-pyrimidin
5-(trans-4-Pentylcyclohexylmethoxy)-2-(3-cyan-4-propanoyl-oxyphenyl)-pyrimidin
2-(trans-4-Pentylcyclohexylmethoxy)-5-(2-fluor-4-pentyl-phenyl)-pyrimidin
2-(trans-4-Pentylcyclohexylmethoxy)-5-(2-chlor-4-pentyl-phenyl)-pyrimidin
2-(trans-4-Pentylcyclohexylmethoxy)-5-(2-brom-4-pentyl-phenyl)-pyrimidin
2-(trans-4-Pentylcyclohexylmethoxy)-5-(2-cyan-4-pentyl-phenyl)-pyrimidin
2-(trans-4-Pentylcyclohexylmethoxy)-5-(2-methyl-4-pentyl-phenyl)-pyrimidin
2-(trans-4-Pentylcyclohexylmethoxy)-5-(3-fluor-4-pentyl-phenyl)-pyrimidin
2-(trans-4-Pentylcyclohexylmethoxy)-5-(3-chlor-4-pentyl-phenyl)-pyrimidin
2-(trans-4-Pentylcyclohexylmethoxy)-5-(3-brom-4-pentyl-phenyl)-pyrimidin
2-(trans-4-Pentylcyclohexylmethoxy)-5-(3-cyan-4-pentyl-phenyl)-pyrimidin
2-(trans-4-Pentylcyclohexylmethoxy)-5-(3-methyl-4-pentyl-phenyl)-pyrimidin
2-(trans-4-Pentylcyclohexylmethoxy)-5-(3-fluor-4-butyloxy-phenyl)-pyrimidin
2-(trans-4-Pentylcyclohexylmethoxy)-5-(3-chlor-4-butyloxy-phenyl)-pyrimidin
2-(trans-4-Pentylcyclohexylmethoxy)-5-(3-brom-4-butyloxy-phenyl)-pyrimidin
2-(trans-4-Pentylcyclohexylmethoxy)-5-(3-cyan-4-butyloxy-phenyl)-pyrimidin
2-(trans-4-Pentylcyclohexylmethoxy)-5-(3-fluor-4-propanoyl-oxyphenyl)-pyrimidin
2-(trans-4-Pentylcyclohexylmethoxy)-5-(3-chlor-4-propanoyl-oxyphenyl)-pyrimidin
2-(trans-4-Pentylcyclohexylmethoxy)-5-(3-brom-4-propanoyl-oxyphenyl)-pyrimidin
2-(trans-4-Pentylcyclohexylmethoxy)-5-(3-cyan-4-propanoyl-oxyphenyl)-pyrimidin
2-Chlor-4-propyl-4'-(4-propylphenyl)terphenyl
2-Clor-3-pentyl-4'-(4-propylphenyl)terphenyl
2-Clor-4-pentyl-4'-(5-pentylphenyl)terphenyl
3-Clor-4-pentyl-4'-(4-pentylphenyl)terphenyl
2-Brom-4-pentyl-4'-(4-pentylphenyl)terphenyl
3-Brom-4-pentyl-4'-(4-pentylphenyl)terphenyl
2-Methyl-4-pentyl-4'-(4-pentylphenyl)terphenyl
3-Methyl-4-pentyl-4'-(4-pentylphenyl)terphenyl
2-Brom-4-butyloxy-4'-(4-pentylphenyl)terphenyl
2-Methyl-4-butyloxy-4'-(4-pentylphenyl)terphenyl
2-Cyan-4-butyloxy-4'-(4-pentylphenyl)terphenyl
2-Chlor-1,4-bis(4',4''-dipentylphenyl)diphenyl
2-Brom-1,4-bis(4',4''-dipentylphenyl)diphenyl
2-Cyan-1,4-bis(4',4''-dipentylphenyl)diphenyl
2-Methyl-1,4-bis(4',4''-dipentylphenyl)diphenyl
1,4-Bis(3-chlor-4-pentylphenyl)diphenyl
1,4-Bis(3-brom-4-pentylphenyl)diphenyl
1,4-Bis(3-methyl-4-pentylphenyl)diphenyl
1-(2-Chlor-4-pentyldiphenyl)-2-(4'-pentyldiphenyl)aethan
1-(2-Brom-4-pentyldiphenyl)-2-(4'-pentyldiphenyl)aethan
1-(2-Cyan-4-pentyldiphenyl)-2-(4'-pentyldiphenyl)aethan
1-(2-Methyl-4-pentyldiphenyl)-2-(4'-pentyldiphenyl)aethan
1-(4'-pentyldiphenyl)-2-[2-brom-4'-trans-pentylcyclohexyl) phenyl]aethan
1-(4-Butyloxy-3-methyldiphenyl)-2-[3-methyl-4-(4'-trans-pentylcyclohexyl)phenyl]aethan
1-(3-Pentyldiphenyl)-2-[2-brom-4-(4'-trans-pentylcyclohexyl-methoxy)-phenyl]aethan
5-(4-Pentyldiphenyl)-2-(2-chlor-4-pentylphenyl)pyrimidin
5-(4-Pentyldiphenyl)-2-(2-brom-4-pentylphenyl)pyrimidin
5-(4-Pentyldiphenyl)-2-(2-methyl-4-pentylphenyl)pyrimidin
5-(4-Pentyldiphenyl)-2-(2-iod-4-pentylphenyl)pyrimidin
5-(3-Chlor-4-Pentyldiphenyl)-2-(2-chlor-4-pentyldiphenyl)-pyrimidin
2-(4-Pentylphenyl)-5-(3'-brom-4'-butyloxydiphenyl)-pyrimidin
2-(4-Pentylphenyl)-5-(3'-iod-4'-butyloxydiphenyl)-pyrimidin
2-(4-Pentylphenyl)-5-(3'-methyl-4'-butyloxydiphenyl-pyrimidin
5-(4-Pentylphenyl)-2-[2-brom-4-(4'-pentylphenylaethyl)-phenyl]pyrimidin
5-(trans-4-Pentylcyclohexyl)-2-(2-brom-4-pentyldiphenyl)-pyrimidin
5-(trans-4-Pentylcyclohexyl)-2-(2-methyl-4-pentyldiphenyl)-pyrimidin
5-(trans-4-Pentylcyclohexyl)-2-(2-cyan-4-pentyldiphenyl)-pyrimidin
5-(trans-4-Pentylcyclohexyl)-2-(2-iod-4-pentyldiphenyl)-pyrimidin

5-(trans-4-Pentylcyclohexyl)-2-(3-chlor-4-butyloxydiphenyl)-pyrimidin
5-(trans-4-Pentylcyclohexyl)-2-(3-brom-4-butyloxydiphenyl)-pyrimidin
5-(trans-4-Pentylcyclohexyl)-2-(3-cyan-4-butyloxydiphenyl)-pyrimidin
5-(trans-4-Pentylcyclohexyl)-2-(3-methyl-4-butyloxydiphenyl)-pyrimidin
5-(trans-4-Pentylcyclohexyl)-2-(3-brom-4-pentyldiphenyl)-pyrimidin
5-(trans-4-Pentylcyclohexyl)-2-(3-cyan-4-pentyldiphenyl)-pyrimidin
5-(trans-4-Pentylcyclohexyl)-2-(3-methyl-4-pentyldiphenyl)-pyrimidin
5-(trans-4-Pentylcyclohexylaethyl)-2-(2-chlor-4-pentyldiphenyl)-pyrimidin
5-(trans-4-Pentylcyclohexylaethyl)-2-(2-brom-4-pentyldiphenyl)-pyrimidin
5-(trans-4-Pentylcyclohexylaethyl)-2-)2-cyan-4-pentyldiphenyl)-pyrimidin
5-(trans-4-Pentylcyclohexylaethyl)-2-)2-methyl-4-pentyldiphenyl)-pyrimidin
2-Cyano-4,4'-dipentyl-diphenyl
3-Cyano-4,4'-dipentyl-diphenyl
3-Cyano-4-butyloxy-4'-pentyldiphenyl, K 35 N (-40) I
2-Cyano-4-(4-trans-pentylcyclohexyl)-butyloxybenzol, Schmelzpunkt 35°
1-(2-Cyano-4-pentylphenyl)-2-(4-pentylphenyl)-ethan
2,3-Dicyano-1,4-bis(4',4''-dipentylphenyl)-benyol
1-Butyloxy-2,3-dicyano-4-(4'-pentylbiphenylyl)-benzol
1-Pentyl-2,3-dicyano-4-(4'-pentylbiphenylyl)-benzol
1-(2,3-Dicyano-4-pentylphenyl)-2-(4'-pentylbiphenylyl)-ethan
1-(2,3-Dicyano-4-butyloxyphenyl)-2-(4'-pentylbiphenylyl)-ethan
1-(2,3-Dicyano-4-butyloxyphenyl)-2-[4-(4'-trans-pentylcyclohexyl)-phenyl]-ethan
1-(2,3-Dicyano-4-pentylphenyl)-2-[4-(4'-trans-pentylcyclohexyl)-phenyl]-ethan
1-(4-Fentylphenyl)-2-[4-(4'-trans-pentylcyclohexyl)-2,3-dicyano-phenyl]-ethan
1-(2,3-Dicyano-4-pentylphenyl)-2-(4,4'-trans,trans-pentyl-bicyclohexyl)-ethan
1-(2,3-Dicyano-4-butyloxyphenyl)-2-(4,4'-trans,trans-pentyl-bicyclohexyl)-ethan
2-Methyl-1-pentyl-4-(4-trans-pentylcyclohexyl)-benzol, $\Delta n = 0{,}08$
3-Methyl-1-pentyl-4-(4-trans-pentylcyclohexyl)-benzol
2-Methyl-1-nonyl-4-(4-trans-pentylcyclohexyl)-benzol
3-Methyl-1-nonyl-4-(4-trans-pentylcyclohexyl)-benzol
2-Methyl-1-nonyl-4-(4-trans-nonylcyclohexyl)-benzol
3-Methyl-1-nonyl-4-(4-trans-nonylcyclohexyl)-benzol
2-Methyl-4-(4-trans-nonylcyclohexyl)-1-pentyl-benzol
3-Methyl-4-(4-trans-nonylcyclohexyl)-1-pentyl-benzol
1-Butoxy-2-methyl-4-(4-trans-pentylcyclohexyl)-benzol
1-Butoxy-3-methyl-4-(4-trans-pentylcyclohexyl)-benzol
1-Butoxy-2-methyl-4-(4-trans-nonylcyclohexyl)-benzol
1-Butoxy-3-methyl-4-(4-trans-nonylcyclohexyl)-benzol
2-Methyl-4-(4-trans-nonylcyclohexyl)-1-octyloxy-benzol
3-Methyl-4-(4-trans-nonylcyclohexyl)-1-octyloxy-benzol
2-Methyl-1-octyloxy-4-(4-trans-pentylcyclohexyl)-benzol
3-Methyl-1-octyloxy-4-(4-trans-pentylcyclohexyl)-benzol
2-Methyl-4-(4-trans-pentylcyclohexyl)-phenyl-butanoat
3-Methyl-4-(4-trans-pentylcyclohexyl)-phenyl-butanoat
2-Methyl-4-(4-trans-nonylcyclohexyl)-phenyl-butanoat
3-Methyl-4-(4-trans-nonylcyclohexyl)-phenyl-butanoat
2-Methyl-4-(4-trans-pentylcyclohexyl)-phenyl-octanoat
3-Methyl-4-(4-trans-pentylcyclohexyl)-phenyl-octanoat
2-Methyl-4-(4-trans-nonylcyclohexyl)-phenyl-octanoat
3-Methyl-4-(4-trans-nonylcyclohexyl)-phenyl-octanoat
N-Butyl-2-methyl-4-(4-trans-pentylcyclohexyl)-anilin
N-Butyl-3-methyl-4-(4-trans-pentylcyclohexyl)-anilin
N-Butyl-2-methyl-4-(4-trans-nonylcyclohexyl)-anilin
N-Butyl-3-methyl-4-(4-trans-nonylcyclohexyl)-anilin
N-Octyl-2-methyl-4-(4-trans-pentylcyclohexyl)-anilin
N-Octyl-3-methyl-4-(4-trans-pentylcyclohexyl)-anilin
N-Octyl-2-methyl-4-(4-trans-nonylcyclohexyl)-anilin
N-Octyl-3-methyl-4-(4-trans-nonylcyclohexyl)-anilin
1-(4-trans-Pentylcyclohexyl)-2-(2-methyl-4-pentylphenyl)-ethan
1-(4-trans-Pentylcyclohexyl)-2-(3-methyl-4-pentylphenyl)-ethan
1-(4-trans-Pentylcyclohexyl)-2-(2-methyl-4-nonylphenyl)-ethan
1-(4-trans-Pentylcyclohexyl)-2-(3-methyl-4-nonylphenyl)-ethan
1-(4-trans-Nonylcyclohexyl)-2-(2-methyl-4-pentylphenyl)-ethan
1-(4-trans-Nonylcyclohexyl)-2-(3-methyl-4-pentylphenyl)-ethan
1-(4-trans-Nonylcyclohexyl)-2-(2-methyl-4-nonylphenyl)-ethan

1-(4-trans-Nonylcyclohexyl)-2-(3-methyl-4-nonylphenyl)-ethan
1-(4-trans-Pentylcyclohexyl)-2-(4-butoxy-2-methylphenyl)-ethan
1-(4-trans-Pentylcyclohexyl)-2-(4-butoxy-3-methylphenyl)-ethan
1-(4-trans-Pentylcyclohexyl)-2-(2-methyl-4-octyloxyphenyl)-ethan
1-(4-trans-Pentylcyclohexyl)-2-(3-methyl-4-octyloxyphenyl)ethan
1-(4-trans-Nonylcyclohexyl)-2-(4-butoxy-2-methylphenyl)-ethan
1-(4-trans-Nonylcyclohexyl)-2-(4-butoxy-3-methylphenyl)-ethan
1-(4-trans-Nonylcyclohexyl)-2-(2-methyl-4-octyloxyphenyl)-ethan
1-(4-trans-Nonylcyclohexyl)-2-(3-methyl-4-octyloxyphenyl)-ethan
1-(4-trans-Pentylcyclohexyl)-2-(4-N-butylamino-2-methyl-phenyl)-ethan
1-(4-trans-Pentylcyclohexyl)-2-(4-N-butylamino-3-methyl-phenyl)-ethan
1-(4-trans-Pentylcyclohexyl)-2-(2-methyl-4-N-octylamino-phenyl)-ethan
1-(4-trans-Pentylcyclohexyl)-2-(3-methyl-4-N-octylamino-phenyl)-ethan
1-(4-trans-Nonylcyclohexyl)-2-(4-N-butylamino-2-methyl-phenyl)-ethan
1-(4-trans-Nonylcyclohexyl)-2-(4-N-butylamino-3-methyl-phenyl)-ethan
1-(4-trans-Nonylcyclohexyl)-2-(2-methyl-4-N-octylamino-phenyl)-ethan
1-(4-trans-Nonylcyclohexyl)-2-(3-methyl-4-N-octylamino-phenyl)-ethan
1-(4-trans-Pentylcyclohexyl)-2-(4-butanoyloxy-2-methyl-phenyl)-ethan
1-(4-trans-Pentylcyclohexyl)-2-(4-butanoyloxy-3-methyl-phenyl)-ethan
1-(4-trans-Pentylcyclohexyl)-2-(2-methyl-4-octanoyloxy-phenyl)-ethan
1-(4-trans-Pentylcyclohexyl)-2-(3-methyl-4-octanoyloxy-phenyl)-ethan
1-(4-trans-Nonylcyclohexyl)-2-(4-butanoyloxy-2-methyl-phenyl)-ethan
1-(4-trans-Nonylcyclohexyl)-2-(4-butanoyloxy-3-methyl-phenyl)-ethan
1-(4-trans-Nonylcyclohexyl)-2-(2-methyl-4-octanoyloxy-phenyl)-ethan
1-(4-trans-Nonylcyclohexyl)-2-(3-methyl-4-octanoyloxy-phenyl)-ethan
(4-trans-Pentylcyclohexylmethyl)-(2-methyl-4-pentyl-phenyl)-äther
(4-trans-Pentylcyclohexylmethyl)-(3-methyl-4-pentyl-phenyl)-äther
(4-trans-Pentylcyclohexylmethyl)-(4-butoxy-2-methyl-phenyl)-äther
(4-trans-Pentylcyclohexylmethyl)-(4-butoxy-3-methyl-phenyl)-äther
(4-trans-Pentylcyclohexylmethyl)-(4-N-butylamino-2-methyl-phenyl)-äther
(4-trans-Pentylcyclohexylmethyl)-(4-N-butylamino-3-methyl-phenyl)-äther
(4-trans-Pentylcyclohexylmethyl)-(2-methyl-4-octanoyloxy-phenyl)-äther
(4-trans-Pentylcyclohexylmethyl)-(3-methyl-4-octanoyloxy-phenyl)-äther
4-(4-trans-Pentylcyclohexyl)-2-methyl-4'-pentyl-biphenyl, $\Delta n = 0{,}16$
4-(4-trans-Pentylcyclohexyl)-3-methyl-4'-pentyl-biphenyl
4-(4-trans-Pentylcyclohexyl)-2-methyl-4'-octanoyloxy-bi-phenyl
4-(4-trans-Pentylcyclohexyl)-3-methyl-4'-octanoyloxy-bi-phenyl
4'-Butoxy-4-(4-trans-pentylcyclohexyl)-2-methyl-biphenyl
4'-Butoxy-4-(4-trans-pentylcyclohexyl)-3-methyl-biphenyl
4'-N-Butylamino-4-(4-trans-pentylcyclohexyl)-2-methyl-bi-phenyl
4'-N-Butylamino-4-(4-trans-pentylcyclohexyl)-3-methyl-bi-phenyl
4-(4-trans-Pentylcyclohexyl)-3,3'-dimethyl-4'-pentyl-bi-phenyl
4-(4-trans-Pentylcyclohexyl)-3,3'-dimethyl-4'-octanoyloxy-biphenyl
4'-N-Butylamino-4-(4-trans-pentylcyclohexyl)-3,3'-dimethyl-biphenyl
4'-Butoxy-4-(4-trans-pentylcyclohexyl)-3,3'-dimethyl-biphenyl
1-(4-(4-trans-Pentylcyclohexyl)-2-methylphenyl)-2-(4-pentyl-phenyl)-ethan
1-(4-(4-trans-Pentylcyclohexyl)-3-methylphenyl)-2-(4-pentyl-phenyl)-ethan
1-(4-(4-trans-Pentylcyclohexyl)-2-methylphenyl)-2-(4-butoxy-phenyl)-ethan
1-(4-(4-trans-Pentylcyclohexyl)-3-methylphenyl)-2-(4-butoxy-phenyl)-ethan
1-(4-(4-trans-Pentylcyclohexyl)-2-methylphenyl)-2-(4-N-butyl-aminophenyl)-ethan
1-(4-(4-trans-Pentylcyclohexyl)-3-methylphenyl)-2-(4-N-butyl-aminophenyl)-ethan
1-(4-(4-trans-Pentylcyclohexyl)-2-methylphenyl)-2-(4-octa-noylphenyl)-ethan
1-(4-(4-trans-Pentylcyclohexyl)-3-methylphenyl)-2-(4-octa-noylphenyl)-ethan
1-(4-(4-trans-Pentylcyclohexyl)-2-methylphenyl)-2-(2-methyl-4-pentylphenyl)-ethan
1-(4-(4-trans-Pentylcyclohexyl)-3-methylphenyl)-2-(2-methyl-4-pentylphenyl)-ethan
1-(4-(4-trans-Pentylcyclohexyl)-2-methylphenyl)-2-(3-methyl-4-pentylphenyl)-ethan
1-(4-(4-trans-Pentylcyclohexyl)-3-methylphenyl)-2-(3-methyl-4-pentylphenyl)-ethan
1-(4-(4-trans-Pentylcyclohexyl)-2-methylphenyl)-2-(4-butoxy-2-methylphenyl)-ethan
1-(4-(4-trans-Pentylcyclohexyl)-3-methylphenyl)-2-(4-butoxy-2-methylphenyl)-ethan
1-(4-(4-trans-Pentylcyclohexyl)-2-methylphenyl)-2-(4-butoxy-3-methylphenyl)-ethan
1-(4-(4-trans-Pentylcyclohexyl)-3-methylphenyl)-2-(4-butoxy-3-methylphenyl)-ethan
1-(4-(4-trans-Pentylcyclohexyl)-2-methylphenyl)-2-(4-N-butyl-amino-2-methylphenyl)-ethan
1-(4-(4-trans-Pentylcyclohexyl)-3-methylphenyl)-2-(4-N-butyl-amino-2-methylphenyl)-ethan
1-(4-(4-trans-Pentylcyclohexyl)-2-methylphenyl)-2-(4-N-butyl-amino-3-methylphenyl)-ethan
1-(4-(4-trans-Pentylcyclohexyl)-3-methylphenyl)-2-(4-N-butyl-amino-3-methylphenyl)-ethan

1-(4-(4-trans-Pentylcyclohexyl)-2-methylphenyl)-2-(2-methyl-4-octanoyloxyphenyl)-ethan
1-(4-(4-trans-Pentylcyclohexyl)-3-methylphenyl)-2-(2-methyl-4-octanoyloxyphenyl)-ethan
1-(4-(4-trans-Pentylcyclohexyl)-2-methylphenyl)-2-(3-methyl-4-octanoyloxyphenyl)-ethan
1-(4-(4-trans-Pentylcyclohexyl)-3-methylphenyl)-2-(3-methyl-4-octanoyloxyphenyl)-ethan
1-(3,3'-Dimethyl-4'-pentylbiphenyl-4-yl)-2-(4-trans-pentyl-cyclohexyl)-ethan
1-(3,3'-Dimethyl-4'-octanoyloxybiphenyl-4-yl)-2-(4-trans-pentylcyclohexyl)-ethan
1-(4'-Butoxy-3,3'-dimethylbiphenyl-4-yl)-2-(4-trans-pentyl-cyclohexyl)-ethan
1-(4'-N-Butylamino-3,3'-dimethylbiphenyl-4-yl)-2-(4-trans-pentylcyclohexyl)-ethan
(3,3'-Dimethyl-4'-pentyl)-biphenyl-4-yl-(4-trans-pentyl-cyclohexyl)-methyl-äther
(3,3'-Dimethyl-4'-octanoyloxy)-biphenyl-4-yl-(4-trans-pen-tylcyclohexyl)-methyl-äther
(4'-Butoxy-3,3'-dimethyl)-biphenyl-4-yl-(4-trans-pentylcyclo-hexyl)-methyl-äther
(4'-N-Butylamino-3,3'-dimethyl)-biphenyl-4-yl-(4-trans-pentyl-cyclohexyl)-methyl-äther
1,4-bis-(4-trans-Propylcyclohexyl)-2-methyl-benzol, $\Delta n = 0{,}11$
1,4-bis-(4-trans-Nonylcyclohexyl)-2-methyl-benzol
1-(4-trans-Nonylcyclohexyl)-4-(4-trans-propylcyclohexyl)-2-methyl-benzol
1-(4-trans-Nonylcyclohexyl)-4-(4-trans-propylcyclohexyl)-3-methyl-benzol
1-(4-trans-Propylcyclohexyl)-2-(4-(4-trans-propylcyclohexyl)-2-methylphenyl)-ethan
1-(4-trans-Propylcyclohexyl)-2-(4-(4-trans-propylcyclohexyl)-3-methylphenyl)-ethan
1-(4-trans-Propylcyclohexyl)-2-(4-(4-trans-nonylcyclohexyl)-2-methylphenyl)-ethan
1-(4-trans-Propylcyclohexyl)-2-(4-(4-trans-nonylcyclohexyl)-3-methylphenyl)-ethan
1-(4-trans-Nonylcyclohexyl)-2-(4-(4-trans-propylcyclohexyl)-2-methylphenyl)-ethan
1-(4-trans-Nonylcyclohexyl)-2-(4-(4-trans-propylcyclohexyl)-3-methylphenyl)-ethan
1-(4-trans-Nonylcyclohexyl)-2-(4-(4-trans-nonylcyclohexyl)-2-methylphenyl)-ethan
1-(4-trans-Nonylcyclohexyl)-2-(4-(4-trans-nonylcyclohexyl)-3-methylphenyl)-ethan
(4-trans-Propylcyclohexyl)-methyl-(4-(4-trans-propylcyclo-hexyl)-2-methyl)-phenyl-äther
(4-trans-Propylcyclohexyl)-methyl-(4-(4-trans-propylcyclo-hexyl)-3-methyl)-phenyl-äther
(4-trans-Propylcyclohexyl)-methyl-(4-(4-trans-nonylcyclo-hexyl)-2-methyl)-phenyl-äther
(4-trans-Propylcyclohexyl)-methyl-(4-(4-trans-nonylcyclo-hexyl)-3-methyl)-phenyl-äther
(4-trans-Nonylcyclohexyl)-methyl-(4-(4-trans-propylcyclo-hexyl)-2-methyl)-phenyl-äther
(4-trans-Nonylcyclohexyl)-methyl-(4-(4-trans-propylcyclo-hexyl)-3-methyl)-phenyl-äther
(4-trans-Nonylcyclohexyl)-methyl-(4-(4-trans-nonylcyclo-hexyl)-2-methyl)-phenyl-äther
(4-trans-Nonylcyclohexyl)-methyl-(4-(4-trans-nonylcyclo-hexyl)-3-methyl)-phenyl-äther
1,4-bis-(2-(4-trans-Propylcyclohexyl)-ethyl)-2-methyl-benzol
1,4-bis-(2-(4-trans-Nonylcyclohexyl)-ethyl)-2-methyl-benzol
1-(2-(4-trans-Nonylcyclohexyl)-ethyl)-4-(2-(4-trans-propyl-cyclohexyl)-ethyl)-2-methyl-benzol
1-(2-(4-trans-Nonylcyclohexyl)-ethyl)-4-(2-(4-trans-propyl-cyclohexyl)-ethyl-3-methyl-benzol
1,4-bis-(2-(4-trans-Propylcyclohexyl)-ethyl)-2,3-dimethyl-benzol
1,4-bis-(2-(4-trans-Nonylcyclohexyl)-ethyl)-2,3-dimethyl-benzol
1-(2-(4-trans-Nonylcyclohexyl)-ethyl)-4-(2-(4-trans-propyl-cyclohexyl)-ethyl)-2,3-dimethyl-benzol
1-(4'-trans-Pentyl-trans-bicyclohex-4-yl)-2-methyl-4-pentyl-benzol
1-(4'-trans-Pentyl-trans-bicyclohex-4-yl)-3-methyl-4-pentyl-benzol
1-(4'-trans-Pentyl-trans-bicyclohex-4-yl)-4-butoxy-2-methyl-benzol
1-(4'-trans-Pentyl-trans-bicyclohex-4-yl)-4-butoxy-3-methyl-benzol
1-(4'-trans-Pentyl-trans-bicyclohex-4-yl)-4-N-butylamino-2-methyl-benzol
1-(4'-trans-Pentyl-trans-bicyclohex-4-yl)-4-N-butylamino-3-methyl-benzol
1-(4'-trans-Pentyl-trans-bicyclohex-4-yl)-2-methyl-4-octa-noyloxy-benzol
1-(4'-trans-Pentyl-trans-bicyclohex-4-yl)-3-methyl-4-octa-noyloxy-benzol
1-(4'-trans-Pentyl-trans-bicyclohex-4-yl)-2-(2-methyl-4-pentylphenyl)-ethan
1-(4'-trans-Pentyl-trans-bicyclohex-4-yl)-2-(3-methyl-4-pentylphenyl)-ethan
1-(4'-trans-Pentyl-trans-bicyclohex-4-yl)-2-(4-butoxy-2-methylphenyl)-ethan
1-(4'-trans-Pentyl-trans-bicyclohex-4-yl)-2-(4-butoxy-3-methylphenyl)-ethan
1-(4 -trans-Pentyl-trans-bicyclohex-4-yl)-2-(4-N-butylamino-2-methylphenyl)-ethan
1-(4'-trans-Pentyl-trans-bicyclohex-4-yl)-2-(4-N-butylamino-3-methylphenyl)-ethan
1-(4'-trans-Pentyl-trans-bicyclohex-4-yl)-2-(2-methyl-4-octa-noyloxyphenyl)-ethan
1-(4'-trans-Pentyl-trans-bicyclohex-4-yl)-2-(3-methyl-4-octa-noyloxyphenyl)-ethan
(4'-trans-Pentyl-trans-bicyclohex-4-yl)-methyl-(2-methyl-4-pentyl)-phenyl-äther
(4'-trans-Pentyl-trans-bicyclohex-4-yl)-methyl-(3-methyl-4-pentyl)-phenyl-äther
(4'-trans-Pentyl-trans-bicyclohex-4-yl)-methyl-(2-methyl-4-octanoyloxy)-phenyl-äther
(4'-trans-Pentyl-trans-bicyclohex-4-yl)-methyl-(3-methyl-4-octanoyloxy)-phenyl-äther
(4'-trans-Pentyl-trans-bicyclohex-4-yl)-methyl-(4-butoxy-2-methyl)-phenyl-äther
(4'-trans-Pentyl-trans-bicyclohex-4-yl)-methyl-(4-butoxy-3-methyl)-phenyl-äther
(4'-trans-Pentyl-trans-bicyclohex-4-yl)-methyl-(4-N-butyl-amino-2-methyl)-phenyl-äther
(4'-trans-Pentyl-trans-bicyclohex-4-yl)-methyl-(4-N-butyl-amino-3-methyl)-phenyl-äther
5-(4-(4-trans-Pentylcyclohexyl)-2-methylphenyl)-2-heptyl-pyrimidin
5-(4-(4-trans-Pentylcyclohexyl)-3-methylphenyl)-2-heptyl-pyrimidin

2-(4-(4-trans-Pentylcyclohexyl)-2-methylphenyl)-5-heptyl-pyrimidin
2-(4-(4-trans-Pentylcyclohexyl)-3-methylphenyl)-5-heptyl-pyrimidin , K 58 N
2-(4-(4-trans-Pentylcyclohexyl)-2-methylphenyl)-5-butoxy-pyrimidin
2-(4-(4-trans-Pentylcyclohexyl)-3-methylphenyl)-5-butoxy-pyrimidin
5-(4-(2-(4-trans-Propylcyclohexyl)-ethyl)-2-methylphenyl)-2-propyl-pyrimidin
5-(4-(2-(4-trans-Propylcyclohexyl)-ethyl)-3-methylphenyl)-2-propyl-pyrimidin
5-(4-(2-(4-trans-Propylcyclohexyl)-ethyl)-2-methylphenyl)-2-nonyl-pyrimidin
5-(4-(2-(4-trans-Propylcyclohexyl)-ethyl)-3-methylphenyl)-2-nonyl-pyrimidin
5-(4-(2-(4-trans-Nonylcyclohexyl)-ethyl)-2-methylphenyl)-2-propyl-pyrimidin
5-(4-(2-(4-trans-Nonylcyclohexyl)-ethyl)-3-methylphenyl)-2-propyl-pyrimidin
5-(4-(2-(4-trans-Nonylcyclohexyl)-ethyl)-2-methylphenyl)-2-nonyl-pyrimidin
5-(4-(2-(4-trans-Nonylcyclohexyl)-ethyl)-3-methylphenyl)-2-nonyl-pyrimidin
5-(4-(4-trans-Propylcyclohexylmethoxy)-2-methylphenyl)-2-propyl-pyrimidin
5-(4-(4-trans-Propylcyclohexylmethoxy)-3-methylphenyl)-2-propyl-pyrimidin
5-(4-(4-trans-Propylcyclohexylmethoxy)-2-methylphenyl)-2-nonyl-pyrimidin
5-(4-(4-trans-Propylcyclohexylmethoxy)-3-methylphenyl)-2-nonyl-pyrimidin
5-(4-(4-trans-Nonylcyclohexylmethoxy)-2-methylphenyl)-2-propyl-pyrimidin
5-(4-(4-trans-Nonylcyclohexylmethoxy)-3-methylphenyl)-2-propyl-pyrimidin
5-(4-(4-trans-Nonylcyclohexylmethoxy)-2-methylphenyl)-2-nonyl-pyrimidin
5-(4-(4-trans-Nonylcyclohexylmethoxy)-3-methylphenyl)-2-nonyl-pyrimidin
2-(4-(2-(4-trans-Propylcyclohexyl)-ethyl)-2-methylphenyl)-5-propyl-pyrimidin
2-(4-(2-(4-trans-Propylcyclohexyl)-ethyl)-3-methylphenyl)-5-propyl-pyrimidin
2-(4-(2-(4-trans-Propylcyclohexyl)-ethyl)-2-methylphenyl)-5-nonyl-pyrimidin
2-(4-(2-(4-trans-Propylcyclohexyl)-ethyl)-3-methylphenyl)-5-nonyl-pyrimidin
2-(4-(2-(4-trans-Propylcyclohexyl)-ethyl)-2-methylphenyl)-5-butoxy-pyrimidin
2-(4-(2-(4-trans-Propylcyclohexyl)-ethyl)-3-methylphenyl)-5-butoxy-pyrimidin
2-(4-(2-(4-trans-Propylcyclohexyl)-ethyl)-2-methylphenyl)-5-octyloxy-pyrimidin
2-(4-(2-(4-trans-Propylcyclohexyl)-ethyl)-3-methylphenyl)-5-octyloxy-pyrimidin
2-(4-(2-(4-trans-Nonylcyclohexyl)-ethyl)-2-methylphenyl)-5-propyl-pyrimidin
2-(4-(2-(4-trans-Nonylcyclohexyl)-ethyl)-3-methylphenyl)-5-propyl-pyrimidin
2-(4-(2-(4-trans-Nonylcyclohexyl)-ethyl)-2-methylphenyl)-5-nonyl-pyrimidin
2-(4-(2-(4-trans-Nonylcyclohexyl)-ethyl)-3-methylphenyl)-5-nonyl-pyrimidin
2-(4-(2-(4-trans-Nonylcyclohexyl)-ethyl)-2-methylphenyl)-5-butoxy-pyrimidin
2-(4-(2-(4-trans-Nonylcyclohexyl)-ethyl)-3-methylphenyl)-5-butoxy-pyrimidin
2-(4-(2-(4-trans-Nonylcyclohexyl)-ethyl)-2-methylphenyl)-5-octyloxy-pyrimidin
2-(4-(2-(4-trans-Nonylcyclohexyl)-ethyl)-3-methylphenyl)-5-octyloxy-pyrimidin
2-(4-(4-trans-Propylcyclohexylmethoxy)-2-methylphenyl)-5-propyl-pyrimidin
2-(4-(4-trans-Propylcyclohexylmethoxy)-3-methylphenyl)-5-propyl-pyrimidin
2-(4-(4-trans-Propylcyclohexylmethoxy)-2-methylphenyl)-5-nonyl-pyrimidin
2-(4-(4-trans-Propylcyclohexylmethoxy)-3-methylphenyl)-5-nonyl-pyrimidin
2-(4-(4-trans-Propylcyclohexylmethoxy)-2-methylphenyl)-5-butoxy-pyrimidin
2-(4-(4-trans-Propylcyclohexylmethoxy)-3-methylphenyl)-5-butoxy-pyrimidin
2-(4-(4-trans-Propylcyclohexylmethoxy)-2-methylphenyl)-5-octyloxy-pyrimidin
2-(4-(4-trans-Propylcyclohexylmethoxy)-3-methylphenyl)-5-octyloxy-pyrimidin
2-(4-(4-trans-Nonylcyclohexylmethoxy)-2-methylphenyl)-5-propyl-pyrimidin
2-(4-(4-trans-Nonylcyclohexylmethoxy)-3-methylphenyl)-5-propyl-pyrimidin
2-(4-(4-trans-Nonylcyclohexylmethoxy)-2-methylphenyl)-5-nonyl-pyrimidin
2-(4-(4-trans-Nonylcyclohexylmethoxy)-3-methylphenyl)-5-nonyl-pyrimidin
2-(4-(4-trans-Nonylcyclohexylmethoxy)-2-methylphenyl)-5-butoxy-pyrimidin
2-(4-(4-trans-Nonylcyclohexylmethoxy)-3-methylphenyl)-5-butoxy-pyrimidin
2-(4-(4-trans-Nonylcyclohexylmethoxy)-2-methylphenyl)-5-octyloxy-pyrimidin
2-(4-(4-trans-Nonylcyclohexylmethoxy)-3-methylphenyl)-5-octyloxy-pyrimidin
4,4'-bis-(4-trans-Propylcyclohexyl)-2-methyl-biphenyl, $\Delta n = 0{,}16$
4,4'-bis-(4-trans-Propylcyclohexyl)-3-methyl-biphenyl
4,4'-bis-(4-trans-Propylcyclohexyl)-3,3'-dimethyl-biphenyl
4,4'-bis-(4-trans-Propylcyclohexyl)-2,3'-dimethyl-biphenyl
4,4'-bis-(4-trans-Nonylcyclohexyl)-2-methyl-biphenyl
4,4'-bis-(4-trans-Nonylcyclohexyl)-3-methyl-biphenyl
4,4'-bis-(4-trans-Nonylcyclohexyl)-2,3'-dimethyl-biphenyl
4,4'-bis-(4-trans-Nonylcyclohexyl)-3,3'-dimethyl-biphenyl
4-(4-trans-Nonylcyclohexyl)-4'-(4-trans-propylcyclohexyl)-2-methyl-biphenyl
4-(4-trans-Nonylcyclohexyl)-4'-(4-trans-propylcyclohexyl)-3-methyl-biphenyl
4-(4-trans-Nonylcyclohexyl)-4'-(4-trans-propylcyclohexyl)-2'-methyl-biphenyl
4-(4-trans-Nonylcyclohexyl)-4'-(4-trans-propylcyclohexyl)-3'-methyl-biphenyl
4-(4-trans-Nonylcyclohexyl)-4'-(4-trans-propylcyclohexyl)-2,3'-dimethyl-biphenyl

4-(4-trans-Nonylcyclohexyl)-4'-(4-trans-propylcyclohexyl)-2',3-dimethyl-biphenyl
4-(4-trans-Nonylcyclohexyl)-4'-(4-trans-propylcyclohexyl)-3,3'-dimethyl-biphenyl
4,4'-bis-(2-(4-trans-Propylcyclohexyl)-ethyl)-2,3'-dimethyl-biphenyl
4,4'-bis-(2-(4-trans-Propylcyclohexyl)-ethyl)-3,3'-dimethyl-biphenyl
4,4'-bis-(2-(4-trans-Nonylcyclohexyl)-ethyl)-2,3'-dimethyl-biphenyl
4,4'-bis-(2-(4-trans-Nonylcyclohexyl)-ethyl)-3,3'-dimethyl-biphenyl
4-(2-(4-trans-Nonylcyclohexyl)-ethyl)-4'-(2-(4-trans-propyl-cyclohexyl)-ethyl)-2,3'-dimethyl-biphenyl
4-(2-(4-trans-Nonylcyclohexyl)-ethyl)-4'-(2-(4-trans-propyl-cyclohexyl)-ethyl)-2',3-dimethyl-biphenyl
4-(2-(4-trans-Nonylcyclohexyl)-ethyl)-4'-(2-(4-trans-propyl-cyclohexyl)-ethyl)-3,3'-dimethyl-biphenyl
4,4'-bis-(4-trans-Propylcyclohexylmethoxy)-2,3'-dimethyl-biphenyl
4,4'-bis-(4-trans-Propylcyclohexylmethoxy)-3,3'-dimethyl-biphenyl
4,4'-bis-(4-trans-Nonylcyclohexylmethoxy)-2,3'-dimethyl-biphenyl
4,4'-bis-(4-trans-Nonylcyclohexylmethoxy)-3,3'-dimethyl-biphenyl
4-(4-trans-Nonylcyclohexylmethoxy)-4'-(4-trans-propylcyclo-hexylmethoxy)-2,3'-dimethyl-biphenyl
4-(4-trans-Nonylcyclohexylmethoxy)-4'-(4-trans-propylcyclo-hexylmethoxy)-2',3-dimethyl-biphenyl
4-(4-trans-Nonylcyclohexylmethoxy)-4'-(4-trans-propylcyclo-hexylmethoxy)-3,3'-dimethyl-biphenyl
4-(4'-trans-Pentyl-trans-bicyclohex-4-yl)-2,3'-dimethyl-4'-pentyl-biphenyl
4-(4'-trans-Pentyl-trans-bicyclohex-4-yl)-2',3-dimethyl-4'-pentyl-biphenyl
4-(4'-trans-Pentyl-trans-bicyclohex-4-yl)-3,3'-dimethyl-4'-pentyl-biphentyl
4-(4'-trans-Pentyl-trans bicyclohex-4-yl)-4'-butoxy-2,3'-dimenthyl-biphenyl
4-(4'-trans-Pentyl-trans-bicyclohex-4-yl)-4'-butoxy-2',3-dimenthyl-biphenyl
4-(4'-trans-Pentyl-trans-bicyclohex-4-yl)-4'-butoxy-3,3'-dimethyl-biphenyl
4-(4 -trans-Pentyl-trans-bicyclohex-4-yl)-4'-N-butylamino-2,3'-dimethyl-biphenyl
4-(4'-trans-Pentyl-trans-bicyclohex-4-yl)-4'-N-butylamino-2',3-dimethyl-biphenyl
4-(4'-trans-Pentyl-trans-bicyclohex-4-yl)-4'-N-butylamino-3,3'-dimethyl-biphenyl
4-(4'-trans-Pentyl-trans-bicyclohex-4-yl)-2,3'-dimethyl-4'-octanoyloxy-biphenyl
4-(4'-trans-Pentyl-trans-bicyclohex-4-yl)-2',3-dimethyl-4'-octanoyloxy-biphenyl
4-(4'-trans-Pentyl-trans-bicyclohex-4-yl)-3,3'-dimethyl-4'-octanoyloxy-biphenyl
4-(4'-trans-Pentyl-trans-bicyclohex-4-yl)-2-methyl-4'-pentyl-biphenyl
4-(4'-trans-Pentyl-trans-bicyclohex-4-yl)-3-methyl-4'-pentyl-biphenyl
4-(4'-trans-Pentyl-trans-bicyclohex-4-yl)-2-methyl-4'-octa noyloxy-biphenyl
4-(4'-trans-Pentyl-trans-bicyclohex-4-yl)-3-methyl-4'-octa-noyloxy-biphenyl
4-(4'-trans-Pentyl-trans-bicyclohex-4-yl)-4'-butoxy-2-methyl-biphenyl
4-(4'-trans-Pentyl-trans-bicyclohex-4-yl)-4'-butoxy-3-methyl-biphenyl
4-(4'-trans-Pentyl-trans-bicyclohex-4-yl)-4'-N-butyloxy-2-methyl-biphenyl
4-(4'-trans-Pentyl-trans-bicyclohex-4-yl)-4'-N-butyloxy-3-methyl-biphenyl
1-(4'-trans-Pentyl-trans-bicyclohex-4-yl)-2-(2-methyl-4'-pentylbiphenyl-4-yl)-ethan
1-(4'-trans-Pentyl-trans-bicyclohex-4-yl)-2-(3-methyl-4'-pentylbiphenyl-4-yl)-ethan
1-(4'-trans-Pentyl-trans-bicyclohex-4-yl)-2-(2,3'-dimethyl-4'-pentylbiphenyl-4-yl)-ethan
1-(4'-trans-Pentyl-trans-bicyclohex-4-yl)-2-(2',3-dimethyl-4'-pentylbiphenyl-4-yl)-ethan
1-(4'-trans-Pentyl-trans-bicyclohex-4-yl)-2-(3,3'-dimethyl-4'-pentylbiphenyl-4-yl)-ethan
1-(4'-trans-Pentyl-trans-bicyclohex-4-yl)-2-(4'-butoxy-2-methylbiphenyl-4-yl)-ethan
1-(4'-trans-Pentyl-trans-bicyclohex-4-yl)-2-(4'-butoxy-3-methylbiphenyl-4-yl)-ethan
1-(4'-trans-Pentyl-trans-bicyclohex-4-yl)-2-(4'-butoxy-2,3'-dimethylbiphenyl-4-yl)-ethan
1-(4'-trans-Pentyl-trans-bicyclohex-4-yl)-2-(4'-butoxy-2',3-dimethylbiphenyl-4-yl)-ethan
1-(4'-trans-Pentyl-trans-bicyclohex-4-yl)-2-(4'-butoxy-3,3'-dimethylbiphenyl-4-yl)-ethan
1-(4'-trans-Pentyl-trans-bicyclohex-4-yl)-2-(4'-N-butyl-amino-2-methylbiphenyl-4-yl)-ethan
1-(4'-trans-Pentyl-trans-bicyclohex-4-yl)-2-(4'-N-butyl-amino-3-methylbiphenyl-4-yl)-ethan
1-(4'-trans-Pentyl-trans-bicyclohex-4-yl)-2-(4'-N-butyl-amino-2,3'-dimethylbiphenyl-4-yl)-ethan
1-(4 -trans-Pentyl-trans-bicyclohex-4-yl)-2-(4'-N-butylamino-2',3-dimethylbiphenyl-4-yl)-ethan
1-(4'-trans-Pentyl-trans-bicyclohex-4-yl)-2-(4'-N-butylamino-3,3'-dimethylbiphenyl-4-yl)-ethan
1-(4'-trans-Pentyl-trans-bicyclohex-4-yl)-2-(2-methyl-4'-octanoyloxybiphenyl-4-yl)-ethan
1-(4'-trans-Pentyl-trans-bicyclohex-4-yl)-2-(3-methyl-4'-octanoyloxybiphenyl-4-yl)-ethan
1-(4'-trans-Pentyl-trans-bicyclohex-4-yl)-2-(2,3'-dimethyl-4'-octanoyloxybiphenyl-4-yl)-ethan
1-(4'-trans-Pentyl-trans-bicyclohex-4-yl)-2-(2',3-dimethyl-4'-octanoyloxybiphenyl-4-yl)-ethan
1-(4'-trans-Pentyl-trans-bicyclohex-4-yl)-2-(3,3'-dimethyl-4'-octanoyloxybiphenyl-4-yl)-ethan
(4'-trans-Pentyl-trans-bicyclohex-4-yl)-methyl-(2-methyl-4'-pentyl)-biphenyl-4-yl-äther
(4'-trans-Pentyl-trans-bicyclohex-4-yl)-methyl-(3-methyl-4'-pentyl)-biphenyl-4-yl-äther
(4'-trans-Pentyl-trans-bicyclohex-4-yl)-methyl-(2,3'-dime-thyl-4'-pentyl)-biphenyl-4-yl-äther
(4'-trans-Pentyl-trans-bicyclohex-4-yl)-methyl-(2',3-dime-thyl-4'-pentyl)-biphenyl-4-yl-äther
(4'-trans-Pentyl-trans-bicyclohex-4-yl)-methyl-(3,3'-dime-thyl-4'-pentyl)-biphenyl-4-yl-äther
(4'-trans-Pentyl-trans-bicyclohex-4-yl)-methyl-(4'-butoxy-2-methyl)-biphenyl-4-yl-äther
(4'-trans-Pentyl-trans-bicyclohex-4-yl)-methyl-(4'-butoxy-3-methyl)-biphenyl-4-yl-äther
(4'-trans-Pentyl-trans-bicyclohex-4-yl)-methyl-(4'-butoxy-2,3'-dimethyl)-biphenyl-4-yl-äther
(4'-trans-Pentyl-trans-bicyclohex-4-yl)-methyl-(4'-butoxy-2',3-dimethyl)-biphenyl-4-yl-äther

(4'-trans-Pentyl-trans-bicyclohex-4-yl)-methyl-(4'-butoxy-3,3'-dimethyl)-biphenyl-4-yl-äther
(4'-trans-Pentyl-trans-bicyclohex-4-yl)-methyl-(4'-N-butyl-amino-2-methyl)-biphenyl-4-yl-äther
(4'-trans-Pentyl-trans-bicyclohex-4-yl)-methyl-(4'-N-butyl-amino-3-methyl)-biphenyl-4-yl-äther
(4'-trans-Pentyl-trans-bicyclohex-4-yl)-methyl-(4'-N-butyl-amino-2,3'-dimethyl)-biphenyl-4-yl-äther
(4'-trans-Pentyl-trans-bicyclohex-4-yl)-methyl-(4'-N-butyl-amino-2',3-dimethyl)-biphenyl-4-yl-äther
(4'-trans-Pentyl-trans-bicyclohex-4-yl)-methyl-(4'-N-butyl-amino-3,3'-dimethyl)-biphenyl-4-yl-äther
(4'-trans-Pentyl-trans-bicyclohex-4-yl)-methyl-(2-methyl-4'-octanoyloxy)-biphenyl-4-yl-äther
(4'-trans-Pentyl-trans-bicyclohex-4-yl)-methyl-(3-methyl-4'-octanoyloxy)-biphenyl-4-yl-äther
(4'-trans-Pentyl-trans-bicyclohex-4-yl)-methyl-(2,3'-dimethyl-4'-octanoyloxy)-biphenyl-4-yl-äther
(4'-trans-Pentyl-trans-bicyclohex-4-yl)-methyl-(2',3-dimethyl-4'-octanoyloxy)-biphenyl-4-yl-äther
(4'-trans-Pentyl-trans-bicyclohex-4-yl)-methyl-(3,3'-dimethyl-4'-octanoyloxy)-biphenyl-4-yl-äther.

**Patentansprüche:**

1. Flüssigkristallmischung, enthaltend mindestens eine anisotrope Verbindung mit nematischer Phase der Formel (1)

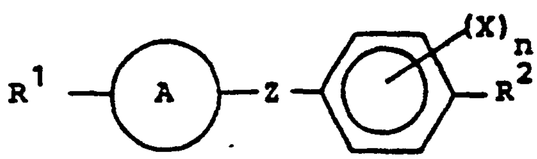

(1)

in der A einen cyclischen Rest gemäß einer der Formeln (10) bis (12)

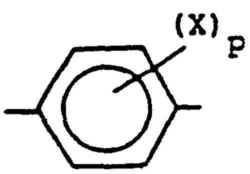

(10)

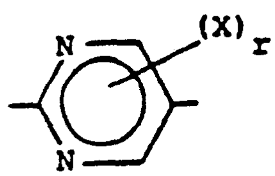

(11)

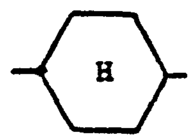

(12)

darstellt, Z die Einfachbindung oder die Gruppe $-CH_2CH_2-$ist, n 1 oder 2, p 0, 1 oder 2, r 0 oder 1 bedeuten, X das Fluor-, Chlor-, Brom- oder Jodatom oder eine Nitril- oder Methylgruppe bedeutet, $R^1$ und $R^2$ Alkyl-, Alkoxy- oder Alkanoyloxy-Gruppen mit jeweils 1 bis 12 C-Atomen im Alkylteil, wobei der Alkylteil gerade oder verzweigt und gegebenenfalls chiral ist, oder cyclische Gruppen der Formeln (13) bis (16)

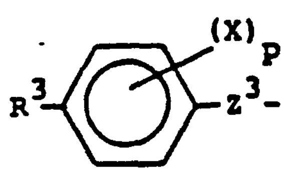

(13)

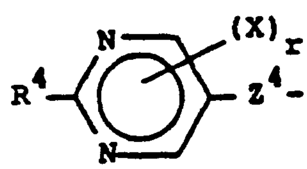

(14)

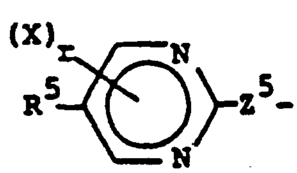

(15)

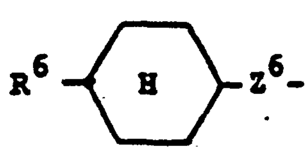

(16)

bedeuten, in welchen $R^3$ bis $R^6$ eine Alkyl-, Alkoxyoder Alkanoyloxy-Gruppe mit jeweils 1 bis 12 C-Atomen im Alkylteil darstellen, wobei der Alkylteil gerade oder verzweigt und gegebenenfalls chiral ist, $Z^2$ bis $Z^6$ eine der für

Z angegebenen Bedeutungen und X, p und r die oben angegebene Bedeutung haben, mit der Maßgabe, daß mindestens ein X im Molekül der Formel (1) Methyl ist, wenn

(A) eine oder beide der Gruppen $R^1$, $R^2$ einen cyclischen Rest der Formel (16) bedeutet, dabei A die Formel (10) hat und die Brückenglieder Z und $Z^6$ die Einfachbindung darstellen , und/oder

(B) nur eine oder keine der Gruppen $R^1$, $R^2$ einen cyclischen Rest der Formeln (13) oder (16) bedeutet, dabei A die Formel (12) hat und das Brückenglied Z sowie das gegebenenfalls vorhandene Brückenglied $Z^3$ oder $Z^6$ die Einfachbindung darstellen.

2. Flüssigkristallmischung nach Anspruch 1, dadurch gekennzeichnet, daß sie eine Verbindung der Formel (22) enthält,

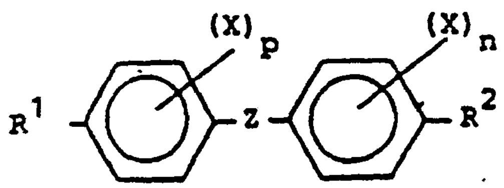

(22)

in der $R^1$, $R^2$, X, Z, p und n die in Anspruch 1 angegebene Bedeutung haben.

3. Flüssigkristallmischung nach Anspruch 1, dadurch gekennzeichnet, daß sie eine Verbindung der Formel (23) enthält,

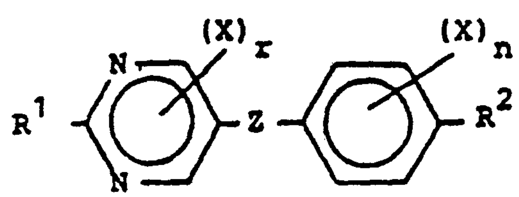

(23)

in der $R^1$, $R^2$, X, Z, n und r die in Anspruch 1 angegebene Bedeutung haben.

4. Flüssigkristallmischung nach Anspruch 1 dadurch gekennzeichnet, daß sie eine Verbindung der Formel (24) enthält

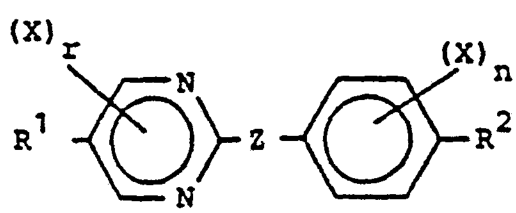

(24)

in der $R^1$, $R^2$, X, Z, r und n die in Anspruch 1 angegebene Bedeutung haben.

5. Flüssigkristallmischung nach Anspruch 1, dadurch gekennzeichnet, daß sie eine Verbindung der Formel (25) enthält,

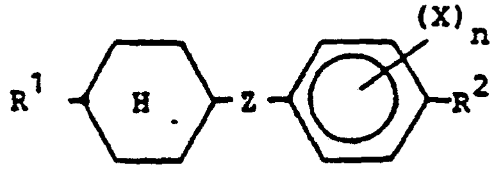

(25)

in der $R^1$, $R^2$, Z, X und n die in Anspruch 1 angegebene Bedeutung haben.

6. Flüssigkristallmischung nach Anspruch 1, dadurch gekennzeichnet, daß sie eine Verbindung der Formel (26) enthält,

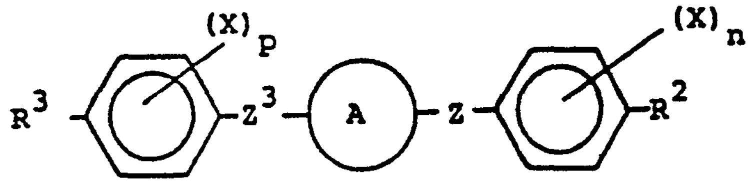

(26)

in der $R^2$, $R^3$, Z, $Z^3$, A, X, p und n die in Anspruch 1 angegebene Bedeutung haben.

7. Flüssigkristallmischung nach Anspruch 1, dadurch gekennzeichnet, daß sie eine Verbindung der Formel (27) enthält,

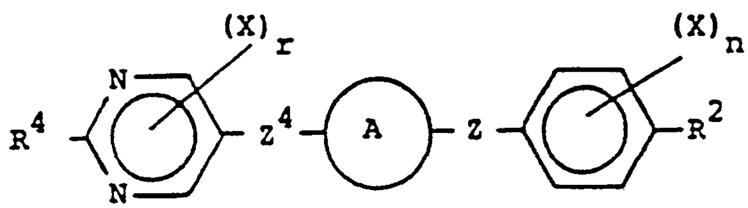

(27)

in der $R^2$, $R^4$, Z, $Z^4$, A, X, n und r die in Anspruch 1 angegebene Bedeutung haben.

8. Flüssigkristallmischung nach Anspruch 1, dadurch gekennzeichnet, daß sie eine Verbindung der Formel (28) enthält,

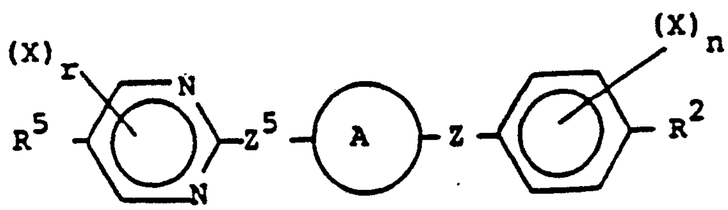

(28)

in der $R^2$, $R^5$, Z, $Z^5$ A X n und r die in Anspruch 1 angegebene Bedeutung haben.

9. Flüssigkristallmischung nach Anspruch 1, dadurch gekennzeichnet, daß sie eine Verbindung der Formel (29) enthält,

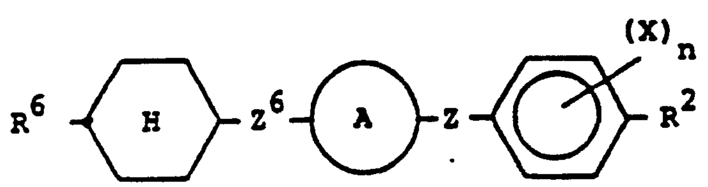

(29)

in der $R^2$, $R^6$ Z $Z^6$ A X und n die in Anspruch 1 angegebene Bedeutung haben.

10. Flüssigkristallmischung nach Anspruch 1, dadurch gekennzeichnet, daß sie eine Verbindung der Formel (30) enthält,

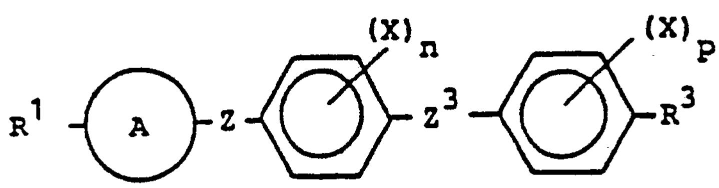

(30)

in der $R^1$, $R^3$, Z, $Z^3$, A, X, n und p die in Anspruch 1 angegebene Bedeutung haben.

11. Flüssigkristallmischung nach Anspruch 1, dadurch gekennzeichnet, daß sie eine Verbindung der Formel (31) enthält,

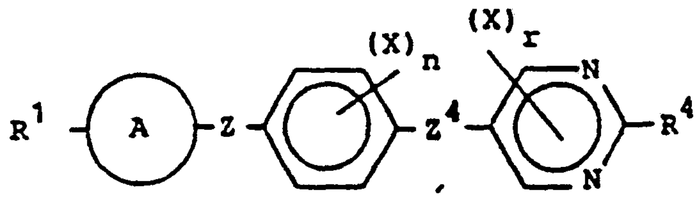

(31)

in der $R^1$ $R^4$ Z $Z^4$ A X, n und r die in Anspruch 1 angegebene Bedeutung haben.

12. Flüssigkristallmischung nach Anspruch 1, dadurch gekennzeichnet, daß sie eine Verbindung der Formel (32) enthält,

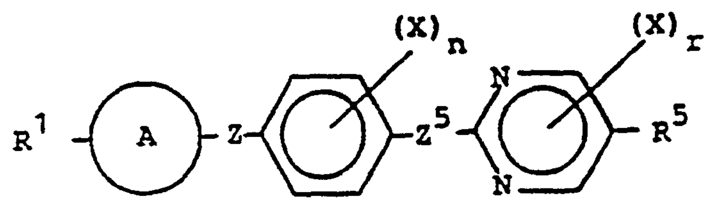

(32)

in der $R^1$, $R^5$, Z, $Z^5$, A, X, n und r die in Anspruch 1 angegebene Bedeutung haben.

13. Flüssigkristallmischung nach Anspruch 1, dadurch gekennzeichnet, daß sie eine Verbindung der Formel (33) enthält,

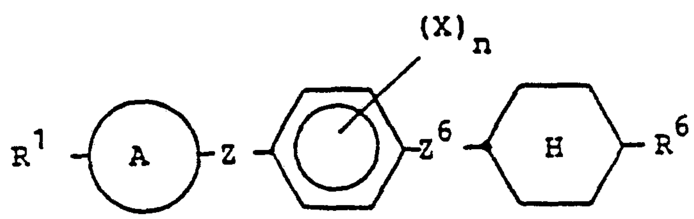

(33)

in der $R^1$, $R^6$, Z, $Z^6$, A, X und n die in Anspruch 1 angegebene Bedeutung haben.

14. Flüssigkristallmischung nach einem der Ansprüche 1 - 13, dadurch gekennzeichnet, daß X die Methylgruppe bedeutet.

15. Flüssigkristallmischung nach einem der Ansprüche 1 - 13, dadurch gekennzeichnet, daß X Fluor, Chlor, Brom oder Jod bedeutet.

## Claims

1. A liquid crystal mixture containing at least one anisotropic compound having a nematic phase having the formula (1)

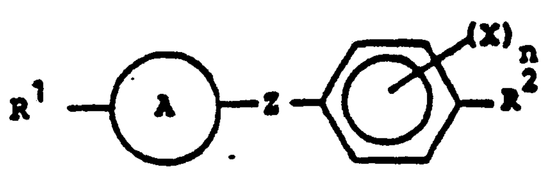

(1)

wherein A is a cyclic radical according to one of the formulae (10) to (12)

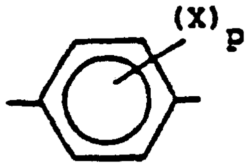

(10)

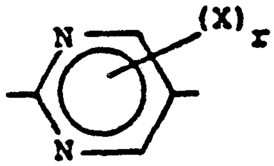

(11)

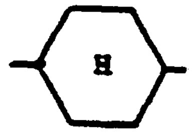

(12)

Z is a single bond or a $-CH_2CH_2-$group,
n is 1 or 2,
p is 0, 1, or 2,
r is 0 or 1,
X is fluorine, chlorine, bromine, iodine, nitrile or methyl,
$R^1$ and $R^2$ are alkyl, alkoxy or alkanoyloxy each having 1 to 12 carbon atoms in the alkyl moiety the alkyl moiety being straight chain or branched and optionally chiral, or are cyclic groups of the formulae (13) to (16)

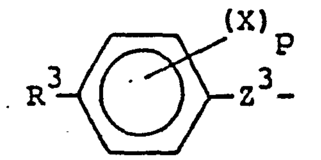

(13)

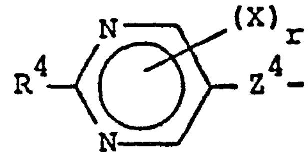

(14)

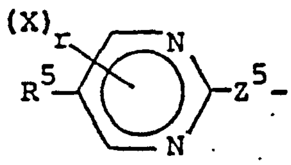

(15)

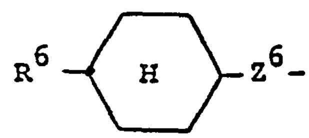

(16)

wherein $R^3$ through $R^6$ are alkyl, alkoxy or alkanoyloxy each having 1 to 12 carbon atoms in the alkyl moiety, the alkyl moiety being straight chain or branched and optionally chiral $Z^2$ to $Z^6$ have one of the meanings given for Z, and X, p and r have the meanings given above, with the proviso that at least one X in the molecule of formula (1) is methyl when

(A) one or none of the groups $R^1$ and $R^2$ is a cyclic radical of the formula (16) A has the formula (10) and the bridging groups Z and $Z^6$ are a single bond and/or

(B) only one or none of the groups $R^1$ and $R^2$ is a cyclic radical of the formula (13) or (16), whereby A has the formula (12) and the bridging group Z and the optionally existing bridging group $Z^3$ or $Z^6$ are a single bond

2. A liquid crystal mixture according to Claim 1 characterised in that it contains a compound having the formula (22)

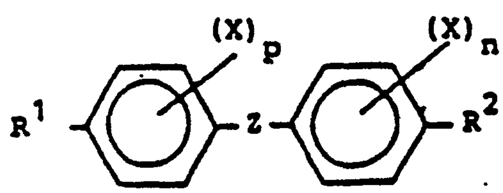

(22)

wherein $R^1$, $R^2$, X, Z, p and n have the meanings given in Claim 1.

3. A liquid crystal mixture according to Claim 1 characterised in that it contains a compound having the formula (23)

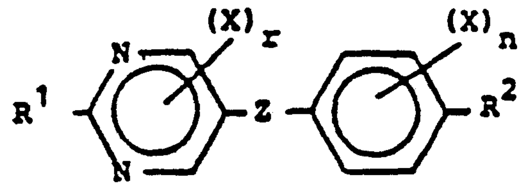

(23)

wherein $R^1$, $R^2$, X, Z, n and r have the meanings given in Claim 1.

4. A liquid crystal mixture according to Claim 1 characterised in that it contains a compound having the formula (24)

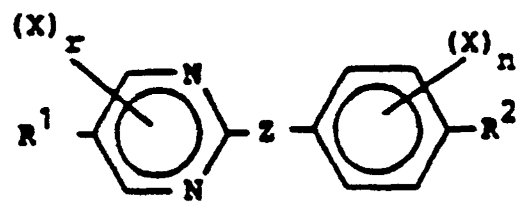

(24)

wherein $R^1$, $R^2$, X, Z, r and n have the meanings given in Claim 1.

5. A liquid crystal mixture according to Claim 1 characterised in that it contains a compound having the formula (25)

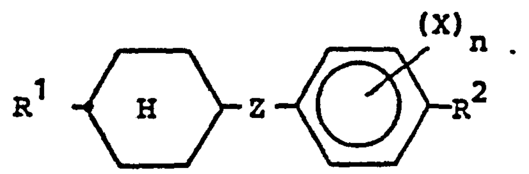

(25)

wherein $R^1$, $R^2$, Z, X and n have the meanings given in Claim 1.

6. A liquid crystal mixture according to Claim 1 characterised in that it contains a compound having the formula (26)

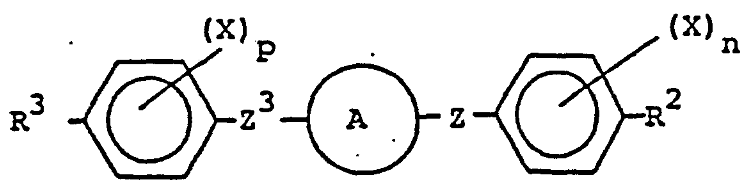

(26)

wherein $R^2$, $R^3$, Z, $Z^3$, A, X, p and n have the meanings given in Claim 1.

7. A liquid crystal mixture according to Claim 1 characterised in that it contains a compound having the formula (27)

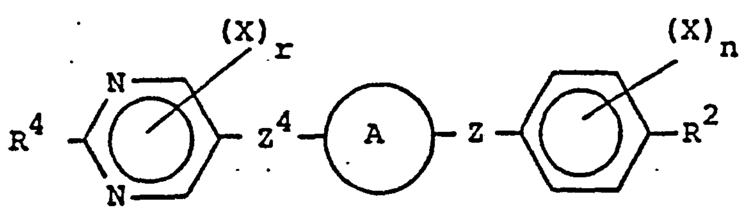

(27)

wherein $R^1$, $R^4$, Z, $Z^4$, A, X, n and r have the meanings given in Claim 1.

8. A liquid crystal mixture according to Claim 1 characterised in that it contains a compounds having the formula (28)

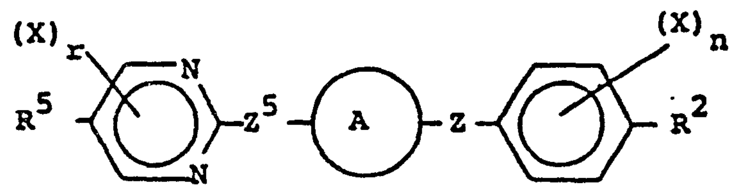

(28)

wherein $R^2$, $R^5$, Z, $Z^5$, A, X, n and r have the meanings given in Claim 1.

9. A liquid crystal mixture according to Claim 1 characterised in that it contains a compound having the formula (29)

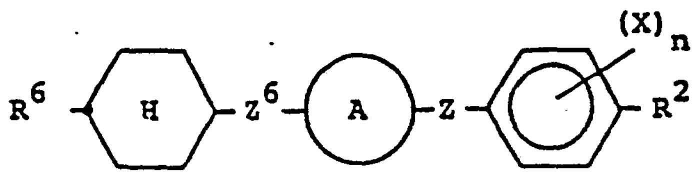

(29)

wherein $R^2$, $R^6$, Z, $Z^6$, A, X and n have the meanings given in Claim 1.

10. A liquid crystal mixture according to Claim 1 characterised in that it contains a compound having the formula (30)

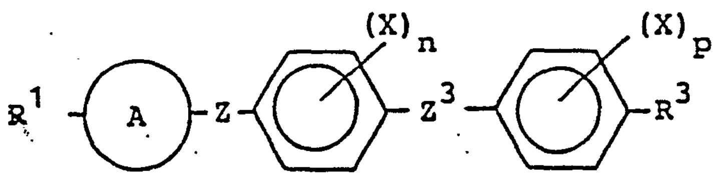

(30)

wherein $R^1$, $R^3$, Z, $Z^3$, A, X, n and p have the meanings given in Claim 1.

11. A liquid crystal mixture according to Claim 1 characterised in that it contains a compound having the formula (31)

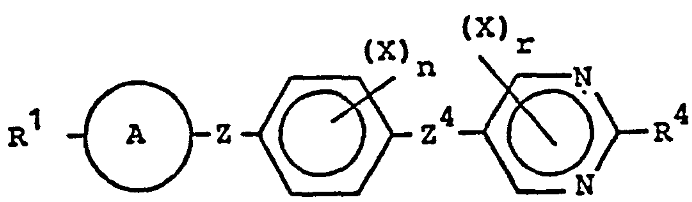

(31)

wherein $R^1$, $R^4$,, Z, $Z^4$, A, X, n and r have the menings given in Claim 1.

12. A liquid crystal mixture according to Claim 1 characterised in that it contains a compound having the formula (32)

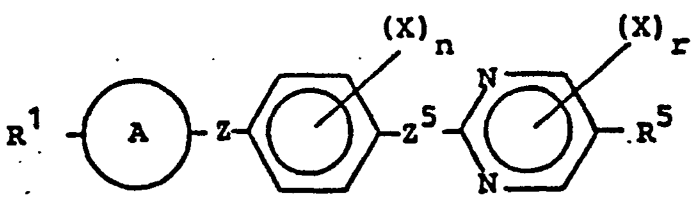

(32)

wherein $R^1$, $R^5$, Z, $Z^5$, A, X, n and r have the meanings given in Claim 1.

13. A liquid crystal mixture according to Claim 1 characterised in that it contains a compound having the formula (33)

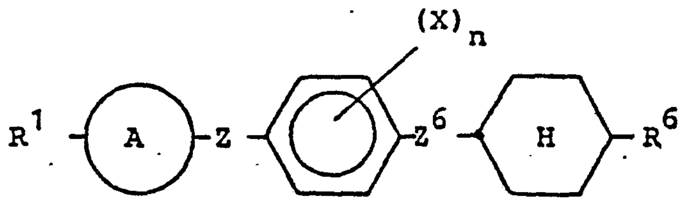

(33)

wherein $R^1$, $R^6$, Z, $Z^6$, A, X and n have the meanings given in Claim 1.

14. A liquid crystal mixture according to one of the Claims 1 - 13, characterised in that X is a methyl group.

15. A liquid crystal mixture according to one of the Claims 1 - 13, characterised in that X is fluorine, chlorine, bromine or jodine.

**Revendications**

1 - Mélange à cristaux liquides, contenant au moins un composé anisotrope à phase nématique de formule 1

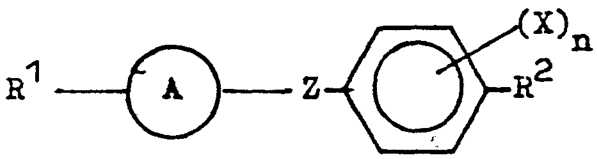

(1)

dans laquelle A représente un reste cyclique répondant à l'une des formules 10 à 12

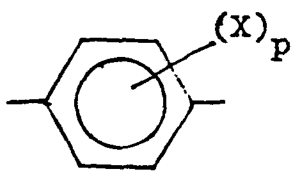 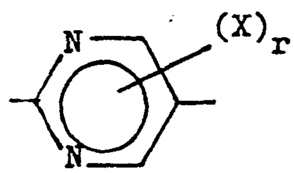 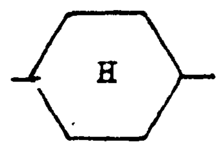

(10) (11) (12)

Z représente une liaison simple ou le groupe $-CH_2CH_2-$, n est egal à 1 ou 2, p est égal à 0, 1 ou 2, r est égal à 0 ou 1, X représente un atome de fluor, de chlore, de brome ou d'iode ou un groupe nitrile ou méthyle, $R^1$ et $R^2$ représentent des groupes alkyle, alcoxy ou alcanoyloxy contenant chacun 1 à 12 atomes de carbone dans le partie alkyle qui est à chaîne droite ou ramifiée et eventuellement chirale, ou des groupes cycliques de formules 13 à 16

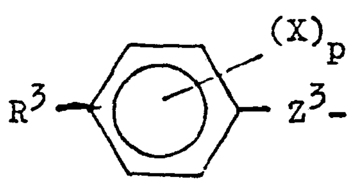 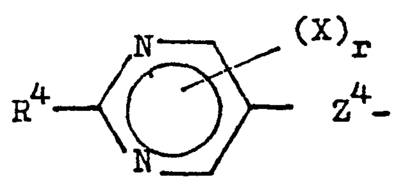

(13) (14)

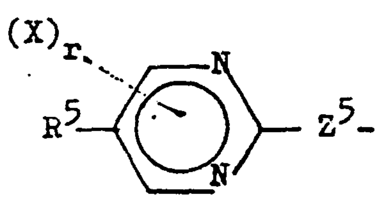 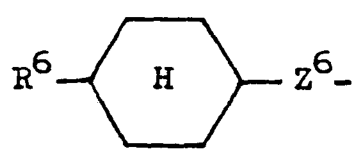

(15) (16)

dans lesquelles $R^3$ à $R^6$ représentent des groupes alkyle alcoxy ou alcanoyloxy contenant chacun 1 à 12 atomes de carbone dans la partie alkyle qui est à chaîne droite ou ramifiée et éventuellement chirale, $Z^2$ à $Z^6$ ont l'une des

significations indiquées pour Z et X, p et r ont les significations indiquées ci-dessus, avec la condition supplémentaire qu'au moins un symbole X de la molécule de formule 1 représente un groupe méthyle lorsque

(A) l'un des groupes $R^1$, $R^2$ ou les deux représentent un reste cyclique de formule 16, A ayant alors la formule 10 et les ponts Z et $Z^6$ consistant en liaisons simples,

et/ou

(B) un seul des symboles $R^1$, $R^2$ ou aucun de ces symboles ne représente un reste cyclique de formule 13 ou 16, A ayant alors la formule 12 et le pont Z ainsi que le pont $Z^3$ ou $Z^6$ éventuellement présent consistant en une liaison simple.

2 - Mélange à cristaux liquides selon la revendication 1, caractérisé en ce qu'il contient un composé de formule 22

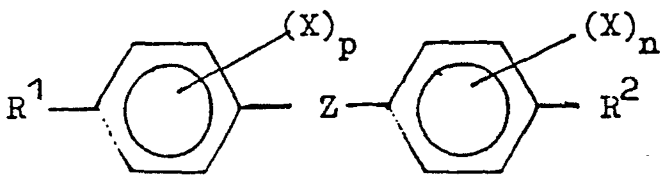

(22)

dans laquelle $R^1$, $R^2$, X, Z, p et n ont les significations indiquées dans la revendication 1.

3 - Mélange à cristaux liquides selon la revendication 1, caractérisé en ce qu'il contient un composé de formule 23

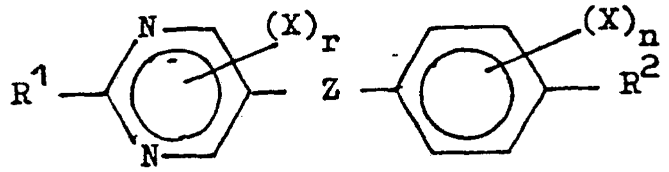

(23)

dans laquelle $R^1$, $R^2$, X, Z, n et r ont les significations indiquées dans la revendication 1.

4 - Mélange à cristaux liquides selon la revendication 1, caractérisé en ce qu'il contient un composé de formule 24

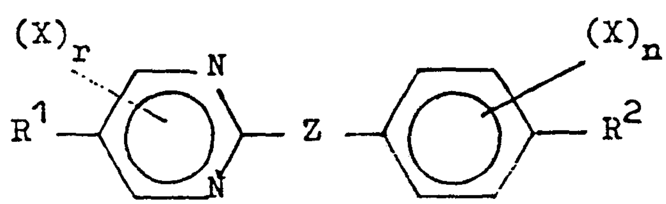

(24)

dans laquelle $R^1$, $R^2$, X, Z, r et n ont les significations indiquées dans la revendication 1.

5 - Mélange à cristaux liquides selon la revendication 1, caractérisé en ce qu'il contient un composé de formule 25

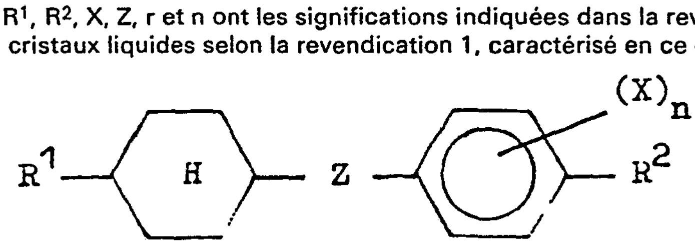

(25)

dans laquelle $R^1$, $R^2$, Z, X et n ont les significations indiquées dans la revendication 1.

6 - Mélange à cristaux liquides selon la revendication 1, caractérisé en ce qu'il contient un composé de formule 26

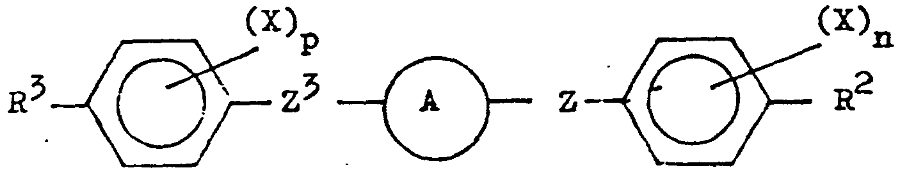 (26)

dans laquelle $R^2$, $R^3$, Z, $Z^3$, A, X, p et n ont les significations indiquées dans la revendication 1.

7 - Mélange à cristaux liquides selon la revendication 1, caractérisé en ce qu'il contient un composé de formule 27

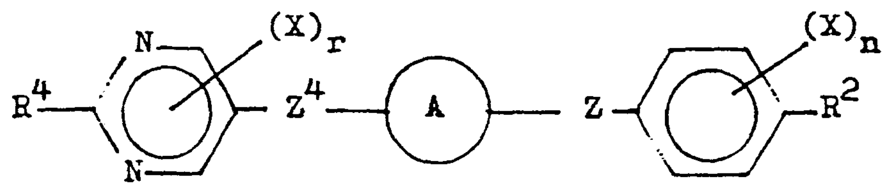 (27)

dans laquelle $R^2$, $R^4$, Z, $Z^4$, A, X, n et r ont les significations indiquées dans la revendication 1.

8 - Mélange à cristaux liquides selon la revendication 1, caractérisé en ce qu'il contient un composé de formule 28

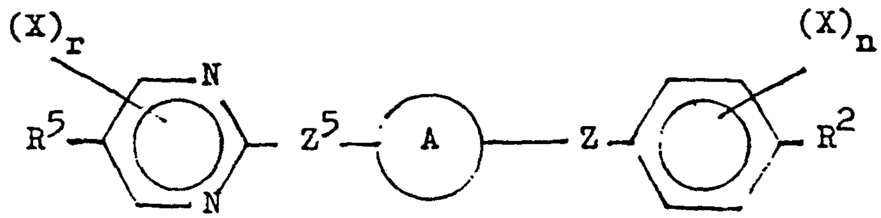 (28)

dans laquelle $R^2$ $R^5$, Z, $Z^5$, A, X, n et r ont les significations indiquées dans la revendication 1.

9 - Mélange à cristaux liquides selon la revendication 1, caractérisé en ce qu'il contient un composé de formule 29

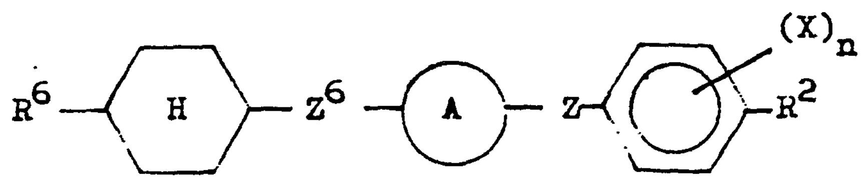 (29)

dans laquelle $R^2$, $R^6$, Z, $Z^6$, A, X et n ont les significations indiquées dans la revendication 1.

10 - Mélange à cristaux liquides selon la revendication 1, caractérisé en ce qu'il contient un composé de formule 30

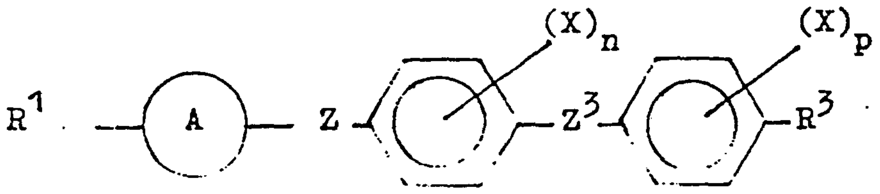

(30)

dans laquelle $R^1$, $R^3$, Z, $Z^3$, A, X, n et p ont les significations indiquées dans la revendication 1.

11. Mélange à cristaux liquides selon la revendication 1, caractérisé en ce qu il contient un composé de formule 31

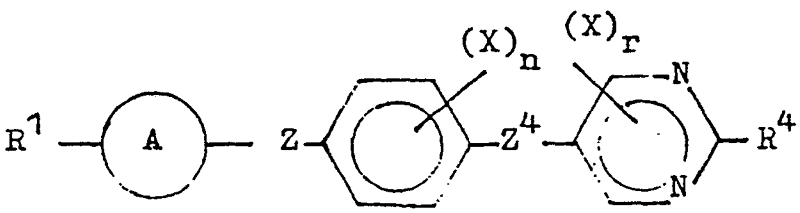

(31)

dans laquelle $R^1$, $R^4$, Z, $Z^4$, A, X n et r ont les significations indiquées dans la revendication 1.

12 - Mélange à cristaux liquides selon la revendication 1, caractérisé en ce qu'il contient un composé de formule 32

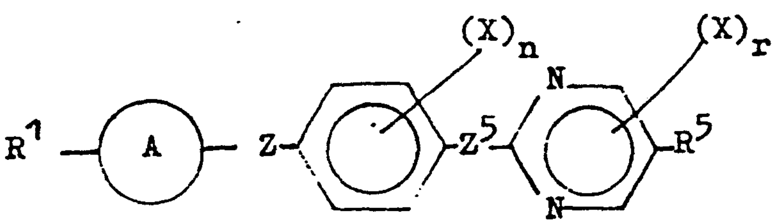

(32)

dans laquelle $R^1$, $R^5$, Z, $Z^5$, A, X, n et r ont les significations indiquées dans la revendication 1.

13 - Mélange à cristaux liquides selon la revendication 1, caractérisé en ce qu'il contient un composé de formule 33

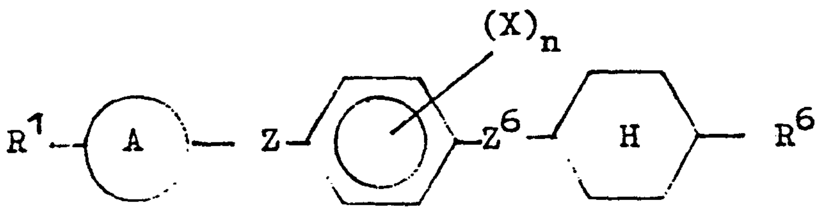

(33)

dans laquelle $R^1$, $R^6$, Z, $Z^6$, A, X et n ont les significations indiquées dans la revendication 1.

14 - Mélange à cristaux liquides selon l'une des revendications 1 à 13, caractérisé en ce que X représente le groupe méthyle.

15 - Mélange à cristaux liquides selon l'une des revendications 1 à 13, caractérisé en ce que X représente le fluor, le chlore, le brome ou l'iode.